# EUROPEAN PATENT APPLICATION

(11) **EP 2 397 157 A1**
(43) Date of publication of application: **21.12.2011**
(21) Application number: 10741251.2
(22) Date of filing: 10.02.2010
(51) Int. Cl.: A61K 45/00, A61K 31/4439, A61K 31/713, A61K 48/00, A61P 35/00, A61P 43/00, C12N 15/09

(54) **BRAIN TUMOR STEM CELL DIFFERENTIATION PROMOTER, AND THERAPEUTIC AGENT FOR BRAIN TUMOR**

(30) Priority: 11.02.2009 US 151645
(71) Applicant: The University of Tokyo, Bunkyo-Ku Tokyo 113-8654 (JP)
(72) Inventor: MIYAZONO, Kohei, Tokyo 113-8654 (JP); IKUSHIMA, Hiroaki, Tokyo 113-8654 (JP); MIYAZAWA, Keiji, Tokyo 113-8654 (JP); TODO, Tomoki, Tokyo 113-8654 (JP); INO, Yasushi, Tokyo 113-8654 (JP)
(74) Representative: Jansen, Cornelis Marinus
(86) International application number: PCT/JP2010/051950
(87) International publication number: WO 2010/092974

(57) **Abstract**

The subject of this invention is to supply a pharmaceutical that can reduce the tumorigenicity of glioma stem cells, which are resistant to anti-cancer drugs and radiation, and also raise sensitivity to anti-cancer drugs and radiation. This invention induces the TGF-β-Sox4-Sox2 pathway (in other words, the TGF-β expresses Sox4) in glioma stem cells, and supplies a differentiation promoter for glioma stem cells, including substances that inhibit the pathway that promotes the expression of Sox2 by Sox4.

## Description

### Technical Fields

This invention concerns a differentiation promoter for brain tumor stem cells, which reduces the tumorigenicity by promoting the differentiation of brain tumor stem cells.

### Background Technology

Brain tumor is the general name for tumors that occur in subcranial tissue, and which occur not only in the brain cells, but in all subcranial tissues, such as the dura, arachnoid membrane, subcranial blood vessels, and peripheral nerves, etc.
Tumors in the brain require treatment that that does not damage as far as possible the normal surrounding tissue. Nevertheless, many brain tumors invade the surrounding brain tissue and spinal tissue to create areas where the tumor cells and normal brain tissue are mixed, so surgical resection is troublesome. Radiotherapy also damages the surrounding normal tissue easily. Further, as the blood-brain barrier exists between the blood and the brain, even if a drip is applied, there are therapeutic agents that do not reach the brain, and chemotherapy might not be effective. Given these facts, a new treatment that is particularly effective on brain tumors is required.

Brain tumors occur in various tissues, so the classification by the World Health Organization (WHO), which uses the origin site as its standard, is used worldwide. Therein, gliomas, which are classified as neuroepithelial tissue tumors, is the general name for tumors that occur in glioma cells. Glioma cells are cells embedded between the nerve cells and nerve fibers in the brain and spinal cord, and gliomas account for approximately one-quarter of tumors occurring in the brain.
Gliomas include astrocytic tumors, oligodendrocytic tumors, and ependymal cell tumors depending on the originating cells, and these are then classified in further detail. In addition, WHO evaluates the clinical malignancy by grades. Grade IV is a highly malignant tumor with poor prognosis, and Grade I is the least malignant. Grade III and Grade IV tumors are called malignant gliomas.
Among gliomas, anaplastic astrocytomas (Grade III) and glioblastomas (Grade IV), which are classified as astrocytic tumors, are particularly highly malignant brain tumors. In particular, glioblastomas are one of those with the poorest prognosis among human cancers, and the five-year survival rate is said to be less than 10%.
As described above, treating brain cancers is generally difficult, and effective treatments for highly-malignant glioblastomas in particular are not to be seen.

In recent years, the inclusion of cells with stem cell-like characteristics in cells that form cancers has become clear, and are called "cancer stem cells" ("CSCs" or "cancer-initiating cells"). CSCs induce tumor formation, and have self-renewal abilities. Recent research has indicated that just a very few CSCs determines the cancer's biological and pathological properties.

In the same way as other tumors, multiple glioma stem cell (GSC) strains are isolated from the human glioma tissue. (See non-patent documents 1 to 3.) GSCs are characterized by expressing neural stem cell antigens, and also by forming non-adhesive spheres called neurospheres or glioma spheres when cultivated in a serum-free culture in the presence of epidermal growth factor (EGF) and basic fibroblast growth factor (bFGF). (See non-patent document 4.)

Cancer treatments to date have targeted the tumor bulk, and it is possible that insufficient effects have been obtained against the cancer stem cells. In reality, there are reports of glioma stem cell resistance to existing radiation and drug treatments in the same way as other cancer stem cells. (See non-patent documents 5 and 6.) Further, cancer stem cells have also been reported as having infiltrative and metastatic abilities. Consequently, it is thought that resecting brain tumor stem cells is necessary in brain tumor treatments, but how the stem cellularity of the brain tumor stem cells is maintained is as yet unclear.

Incidentally, transforming growth factor (TGF)-β is well-known as a cancer suppressant that suppresses the growth of certain types of cancer cells. Nevertheless, TGF-β promotes cancer growth from non-epithelial cells such as brain tumors and osteosarcomas, etc., via PDGF-BB induction. (See non-patent documents 7 and 8.) TGF-β binds to the serine-threonine kinase I and II receptors, and mainly transmits signals within the cell via SMAD proteins. When receptor I phosphatases, the receptor-regulated SMAD (R-SMAD) forms a complex with the common-partner SMAD (co-SMAD) to migrate into the nucleus and regulate the expression of various marker genes. In addition to inducing growth, TGF-β signals are thought to be involved with the infiltration and metastasis of brain tumors, vascularization within tumors, and immunosuppression.

Glioma therapeutic agents that block the TGF-β signals have been developed based on the multiple roles of TGF-β in such tumors. (See non-patent document 9.) Further, clinical research is proceeding by targeting high-grade recurrent or incurable gliomas using TGF-β2-specific anti-sense oligonucleotides. (See non-patent document 10.)

### Advanced Technology Documents

### Non-Patent Documents

Non-patent document 1: Singh et al., Nature, 2004, 432: 396-401
Non-patent document 2: Kondo et al., Proceedings of the National Academy of Sciences USA, 2004, 101: 781-786
Non-patent document 3: Hirschmann-Jax et al., Proceedings of the National Academy of Sciences USA, 2004, 101: 14228-14233
Non-patent document 4: Vescovi et al., Cancer, 2006, 6: 425-436
Non-patent document 5: Bao et al., Nature, 2006, 444: 756-760
Non-patent document 6: Liu et al., Cancer, 2006, 5: 67
Non-patent document 7: Bruna et al., Cancer Cell, 2007, 11: 147-160
Non-patent document 8: Matsuyama et al., Cancer Research, 2003, 63: 7791-7798
Non-patent document 9: Golestaneh and Mishra, Oncogene, 2005, 24: 5722-5730
Non-patent document 10: Schlingensiepen et al., Cytokine Growth Factor Review, 2006,17:129-139

### Summary of the Invention

### Issues the Invention Attempts to Resolve

The subject of this invention is to supply a pharmaceutical that can reduce the tumorigenicity of brain tumor stem cells, and also raise sensitivity to anti-cancer drugs and radiation.

### Methods for Resolving the Issues

The inventors discovered the important effects of the TGF-β pathway, which was hitherto unknown, in maintaining the stem cellularity of brain tumor stem cells as a results of combined research to resolve the issues described above. Namely, they verified that TGF-β raises levels of Sox4 expression, and that the stem cellularity of brain tumor stem cells is maintained by Sox4 raising the expression levels of Sox2, which is a stemness gene.
In addition, they verified that brain tumor stem cell differentiation is promoted by impeding either the functions or the expression of Sox2, Sox4, TGF-β, SMAD2, SMAD3, and Oct-4, which are involved either directly or indirectly in the TGF-β pathway, and thus not only reduces the cell tumorigenicity remarkably, but also raises the sensitivity to anti-cancer drugs and radiation, leading to the completion of this invention.
In other words, this invention involves the following:
(1) A promoter of brain tumor stem cell differentiation, including substances that inhibit the TGF-β-Sox4-Sox2 pathway;
(2) A promoter of brain tumor stem cell differentiation described in (1) above, which is an expression inhibitor or function inhibitor of at least one substance that inhibits the TGF-β-Sox4-Sox2 pathway described above selected from the group comprising Sox2, Sox4, TGF-β, SMAD2, SMAD3, and Oct-4;
(3) A promoter of brain tumor stem cell differentiation described in (2) above, in which the expression inhibitor or function inhibitor described above is a TGF-β receptor antagonist;
(4) A promoter of brain tumor stem cell differentiation described in (2) above, in which the expression inhibitor or function inhibitor described above is a compound selected from the group comprising (a) to (d) below;
   (a) An antisense nucleic acid for at least one selected from the group comprising Sox2, Sox4, SMAD2, SMAD3, and Oct-4
   (b) A nucleic acid with ribozyme activity for at least one selected from the group comprising Sox2, Sox4, SMAD2, SMAD3, and Oct-4
   (c) A double-stranded nucleic acid with RNAi effects for at least one selected from the group comprising Sox2, Sox4, SMAD2, SMAD3, and Oct-4
   (d) A nucleic acid that codes the nucleic acids described in either (a) or (c) above
(5) A promoter of brain tumor stem cell differentiation described in either (1) or (4) above, which is a glioma stem cell differentiation promoter;
(6) A brain tumor therapeutic agent, including a brain tumor stem cell differentiation promoter described in either (1) or (4) above;
(7) A brain tumor therapeutic agent described in (6) above, which is a glioma therapeutic agent;
(8) A double-stranded nucleic acid with RNAi effects on Sox2, comprising two nucleic acid strands described in either (i) or (ii) below;
   (i) A nucleic acid comprising the base sequence described in Sequence Number: 1, and a nucleic acid comprising a complementary base sequence
   (ii) A nucleic acid comprising the base sequence described in Sequence Number: 2, and a nucleic acid comprising a complementary base sequence
(9) A double-stranded nucleic acid with RNAi effects on Sox4 comprising a double-stranded nucleic acid described in either (iii) or (iv) below;
   (iii) A nucleic acid comprising the base sequence described in Sequence Number: 3, and a nucleic acid comprising a complementary base sequence
   (iv) A nucleic acid comprising the base sequence described in Sequence Number: 4, and a nucleic acid comprising a complementary base sequence
(10) A nucleic acid comprising the base sequence described in Sequence Number: 5, and a double-stranded nucleic acid with RNAi effects on SMAD2 comprising a nucleic acid from a complementary base sequence;
(11) A nucleic acid comprising the base sequence described in Sequence Number: 6, and a double-stranded nucleic acid with RNAi effects on SMAD3 comprising a nucleic acid from a complementary base sequence;
(12) A double-stranded nucleic acid with RNAi effects on Oct-4 comprising a double-stranded nucleic acid described in either (v) or (vi) below;
   (v) A nucleic acid comprising the base sequence described in Sequence Number: 51, and a nucleic acid comprising a complementary base sequence
   (vi) A nucleic acid comprising the base sequence described in Sequence Number: 52, and a nucleic acid comprising a complementary base sequence
(13) A treatment method for brain tumors including a process to measure the expression levels of Sox2 and/or Sox4 in the relevant tissues by sampling tissue from the diseased part of the brain tumor of the patient and, if the expression levels of the Sox2 and/or Sox4 described above are elevated compared to normal tissue, a process to administer therapeutic agents for the brain tumor described in (6) or (7) above;
(14) A screening method to determine the treatment method for brain tumor patients, and a method including a process to measure the expression levels of Sox2 and/or Sox4 in the relevant tissues by sampling tissue from the diseased part of the brain tumor of the patient, and a process to compare the expression levels described above to the expression levels in normal tissue.

### Effects of the Invention

The diffusion promoter of the brain tumor stem cells in this invention can strikingly reduce tumorigenicity and increase sensitivity to anti-cancer drugs and radiation by promoting differentiation by directly affecting the brain tumor stem cells reported to have shown resistance to existing anti-cancer drugs and radiation. Consequently, infiltration and metastasis of the brain tumor stem cells can be prevented, and cancer recurrence and metastasis suppressed. Further, there is no malign effect on the surrounding normal brain tissue, and so it is basically possible to treat the brain tumor only.

### Simple Explanation of the Diagrams

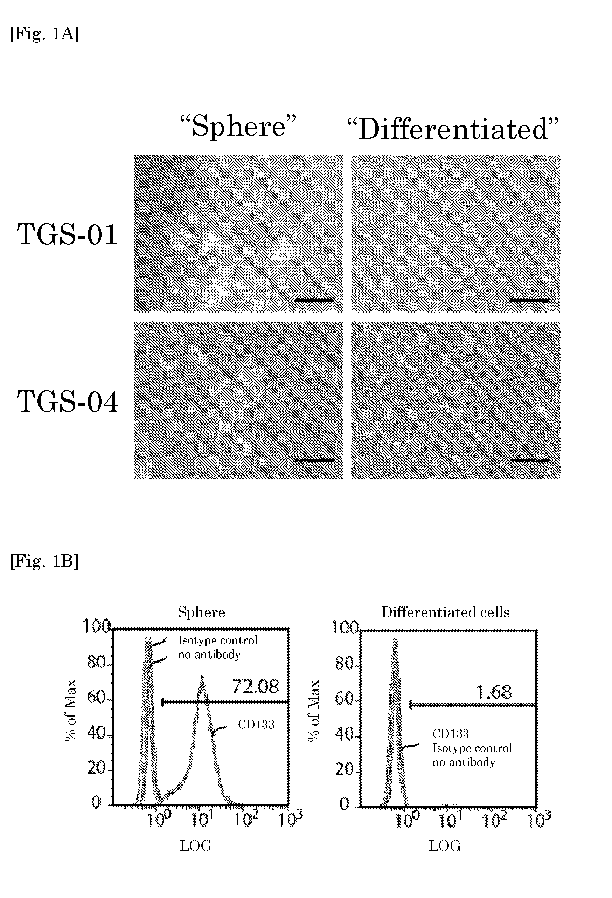

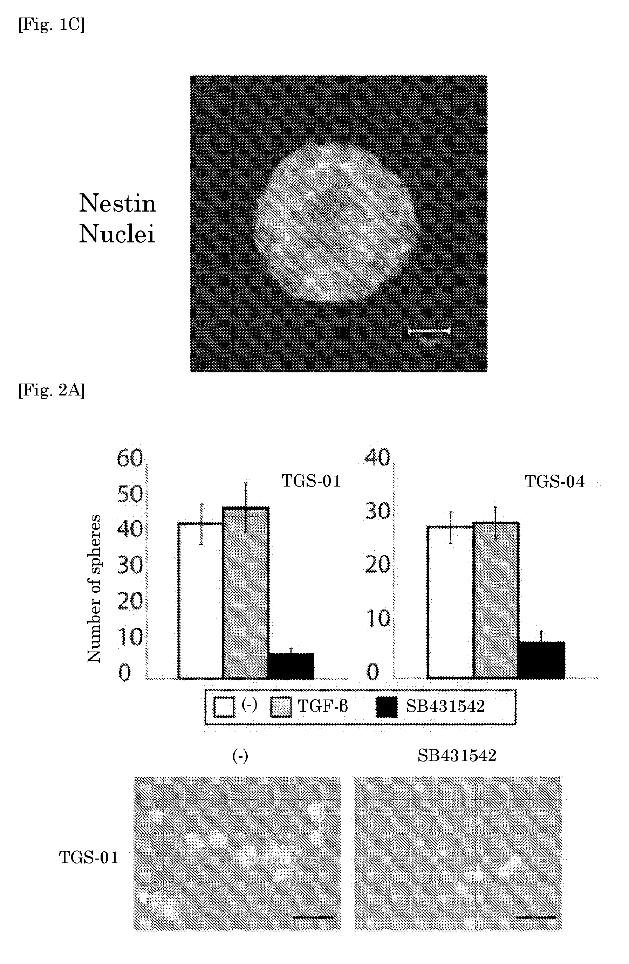

(Fig. 1.) Fig. 1A is a photo of glioma spheres and differentiated cells. The scale bar is 100 µm. Fig. 1B is the results of measuring the CD133-positive cells in the glioma spheres and differentiated cells using flow cytometry. Fig. 1C is a photo showing the expression of the neural precursor cell marker Nestin in the glioma spheres. The scale bar is 20 µm.

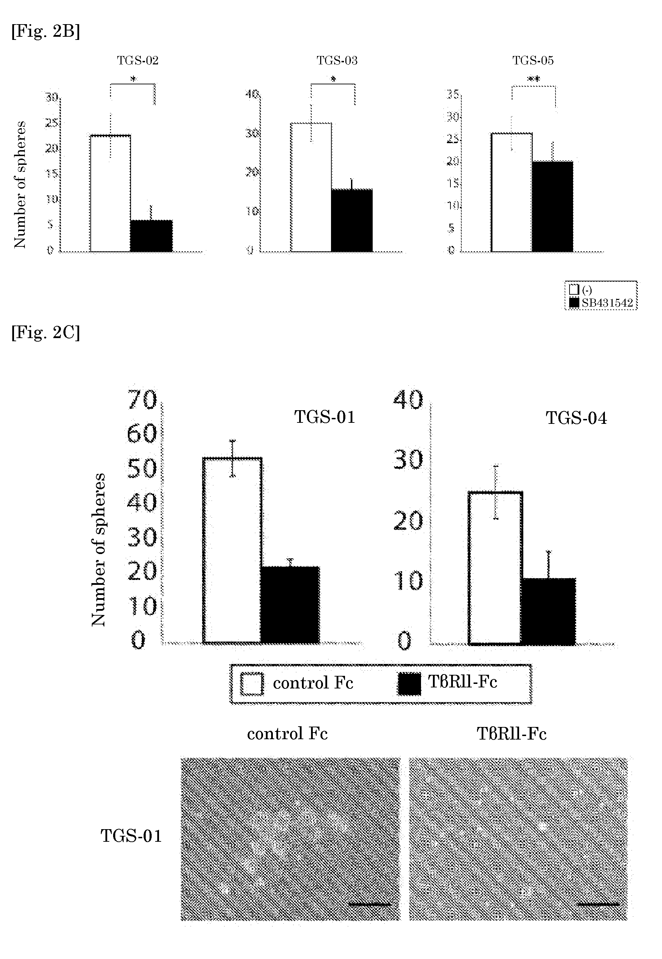

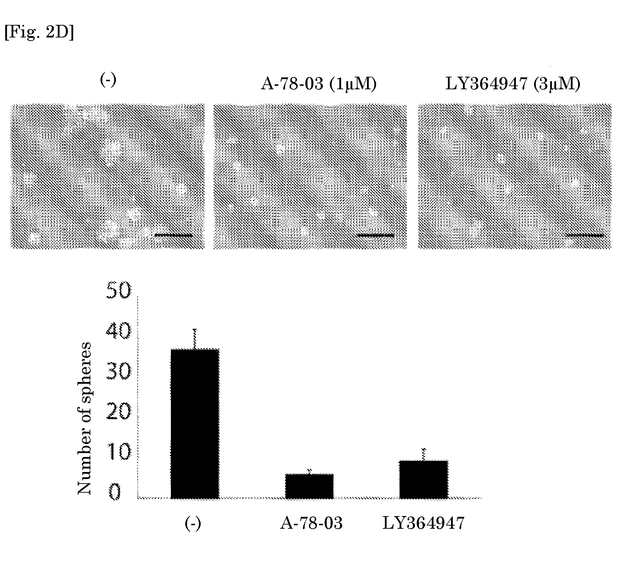

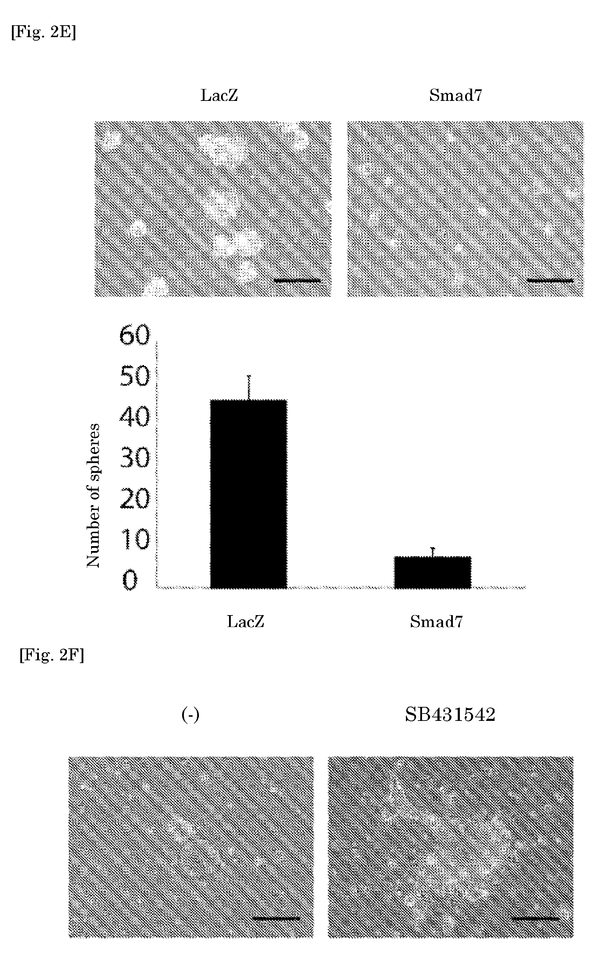

(Fig. 2.) Fig. 2A and Fig. 2B are the results of a sphere formation assay in the presence of the TGF-β inhibitor SB431542. The scale bar is 100 µm. Fig. 2C is the result of a sphere formation assay in the presence of a TGF-β receptor II/Fc chimera. The scale bar is 100 µm. Fig. 2D is the result of a sphere formation assay in the presence of the TGF-β inhibitor A-78-03 or LY364947. Fig. 2E is the result of a sphere formation assay in the presence of the TGF-β signal-negative regulatory factor SMAD7. Fig. 2F is a photo showing that the spheres, once formed, become adhesion cells when SB431542 is added.

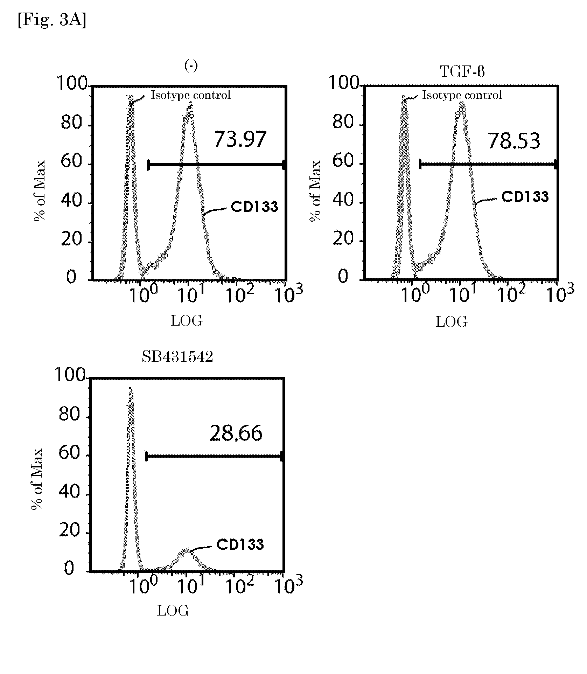

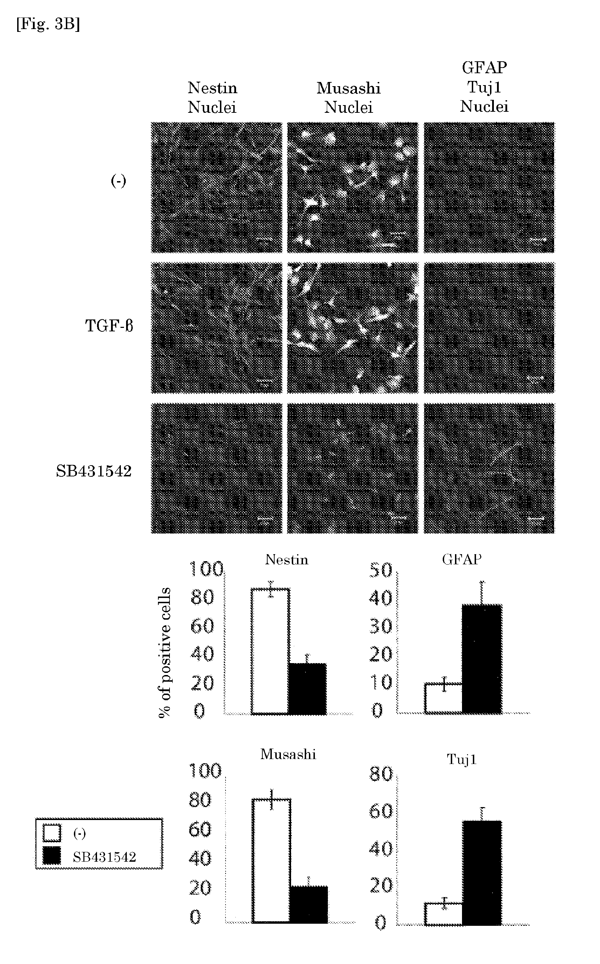

(Fig. 3.) Fig. 3A is the result of examining the impact of a TGF-β inhibitor on the percentage of CD133-positive cells in the glioma spheres. Fig. 3B is the result of examining the expression of neural precursor cell markers or nerve cell differentiation markers in the glioma spheres in the presence of a TGF-β inhibitor.

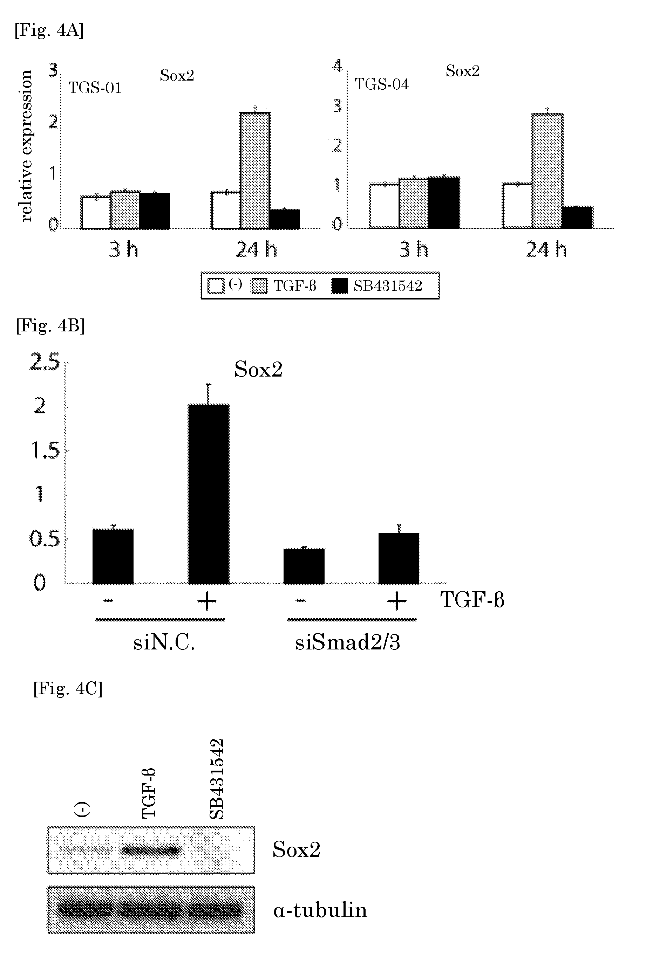

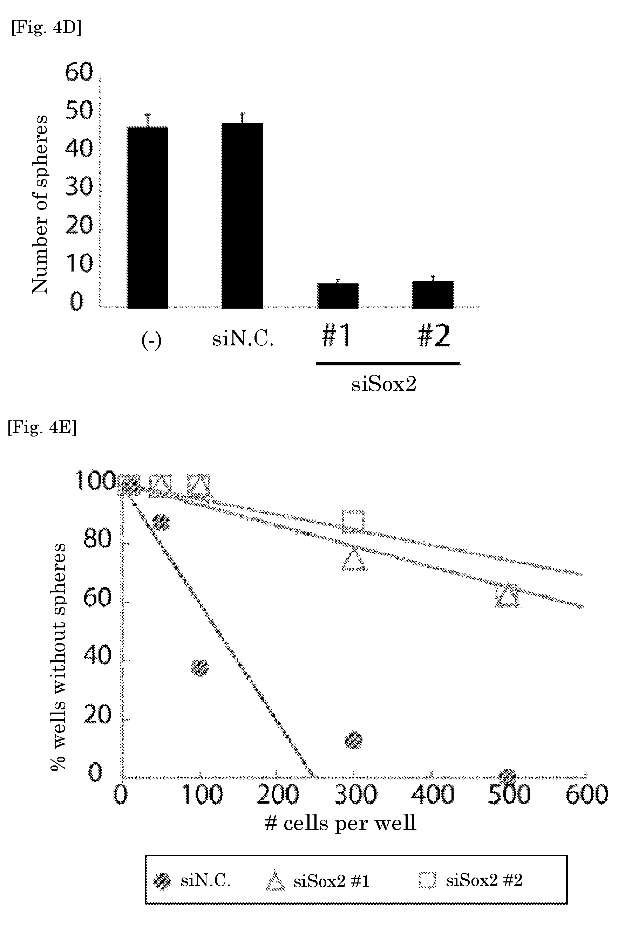

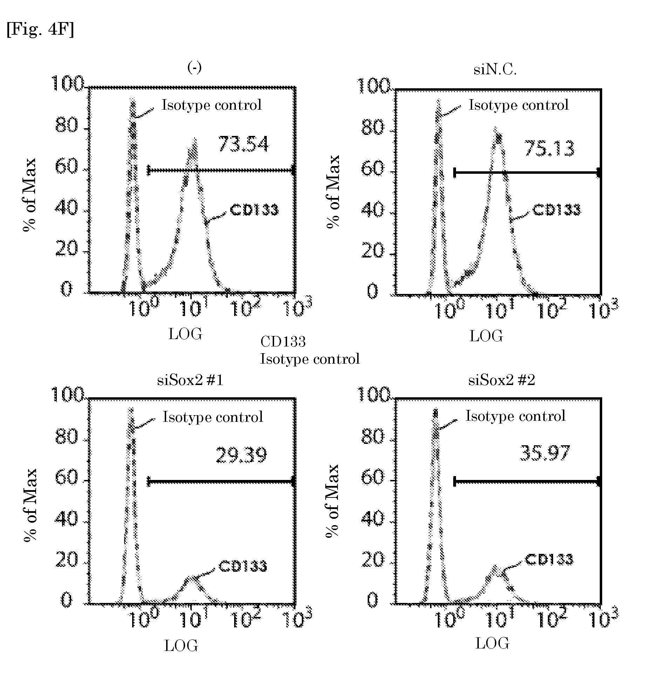

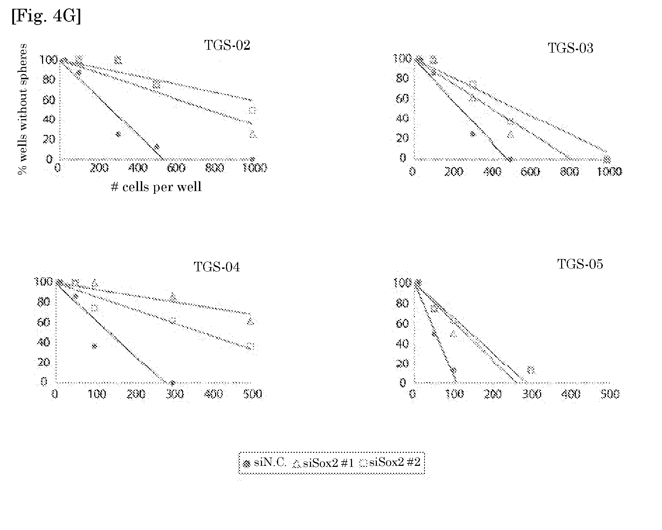

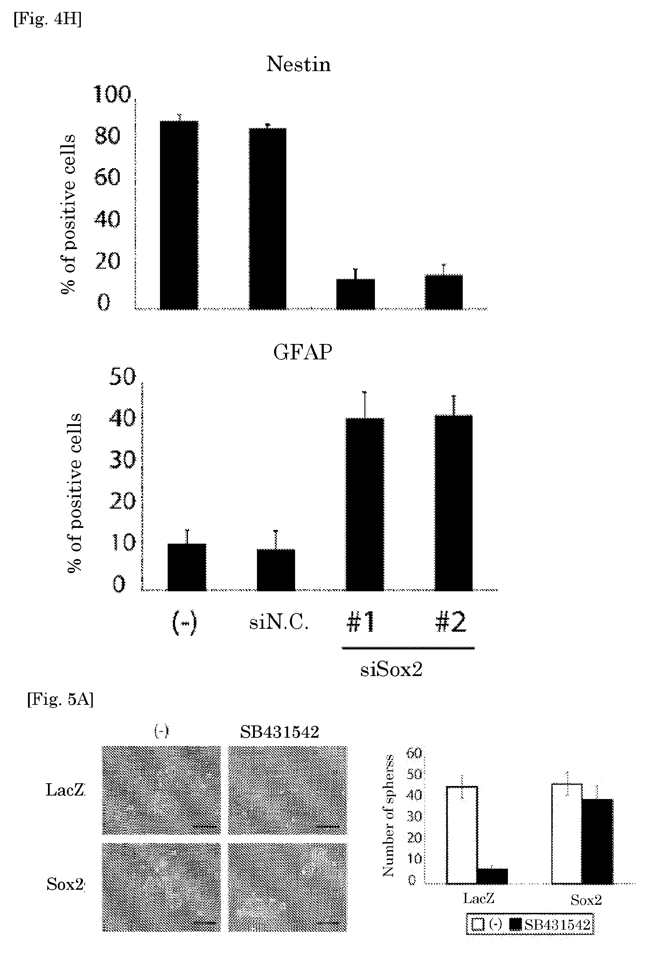

(Fig. 4.) Fig. 4A is the result of measuring the impact of TGF-β or SB431542 on the expression of Sox2 using quantitative real-time RT-PCR. Fig. 4B is the result of examining the impact on SMAD2 and SMAD3 by siRNA, and on Sox2 expression induction by TGF-β. Fig. 4C is the result of measuring the expression of Sox2 at the protein level in the presence of TGF-β or SB431542. Fig. 4D is the result of measuring the impact of Sox2 knockdown by siRNA on the sphere formation abilities of glioma stem cells. Fig. 4E and Fig. 4G are the results of the impact of Sox2 knockdown by siRNA on glioma stem cell self-renewal ability measured using limiting dilution assay. Fig. 4F is the result of the impact of Sox2 knockdown by siRNA on the percentage of CD133-positive cells in the glioma spheres measured using flow cytometry. Fig. 4H is the result of examining the impact of Sox2 knockdown by siRNA on the percentage of Nestin-positive cells and GFAP-positive cells among all the cells.

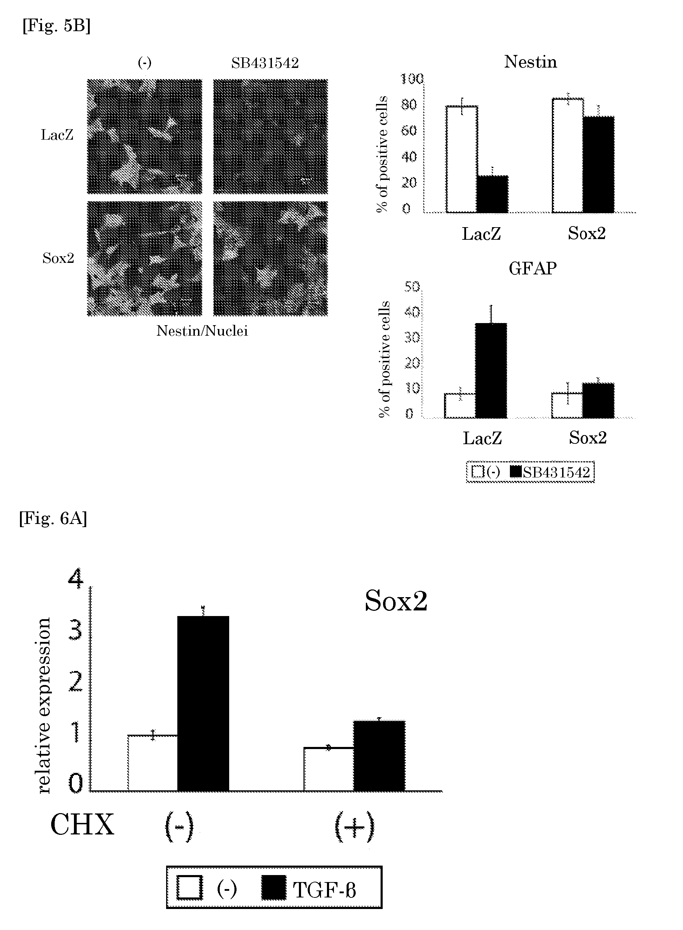

(Fig. 5.) Fig. 5A is the result of measuring the sphere formation ability of the glioma stem cells that expressed excessive Sox2 using sphere formation ability assay in the presence or absence of a TGF-β inhibitor. Fig. 5B is the result of measuring the percentage of Nestin-positive cells or GFAP-positive cells in the glioma spheres that expressed excessive Sox2.

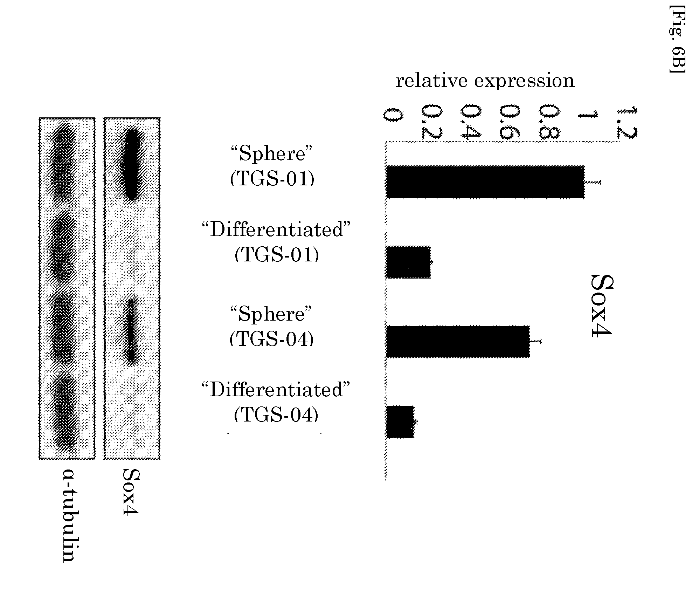

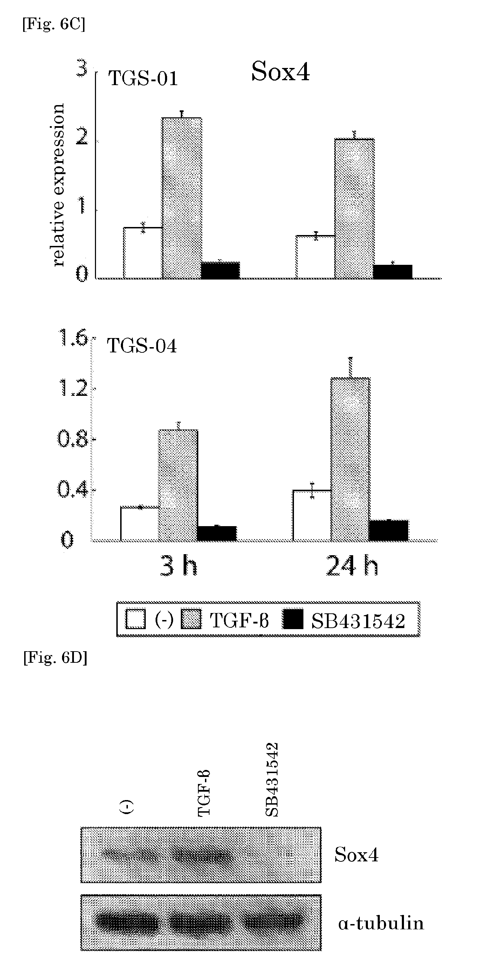

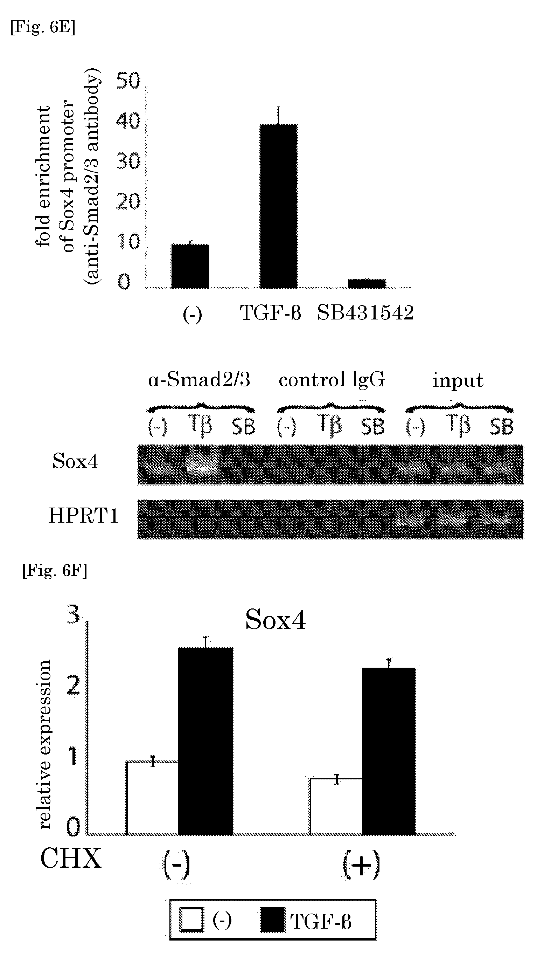

(Fig. 6.) Fig. 6A is the result of examining the impact of TGF-β on Sox2 expression in the presence of cycloheximide (CHX). Fig. 6B is the result of measuring the Sox4 expression levels in glioma stem cells and differentiated glioma cells. Fig. 6C is the result of measuring the mRNA expression level of Sox4 in the presence of TGF-β or a TGF-β inhibitor. Fig. 6D is the result of measuring Sox4 proteins in the presence of TGF-β or a TGF-β inhibitor. Fig. 6E is the result of a chromatin immunoprecipitation assay using SMAD2 and SMAD3 antibodies, which was performed to verify whether or not SMAD complexes, which are DNA-binding signal mediators for TGF-β signals, bind to Sox4 promoters. Fig. 6F is the result of examining the impact of TGF-β on the expression of Sox4 in the presence of cycloheximide.

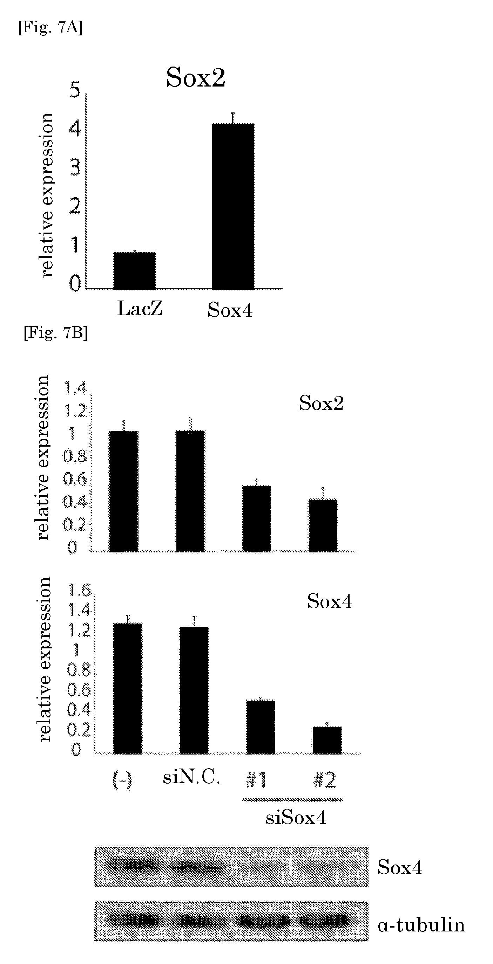

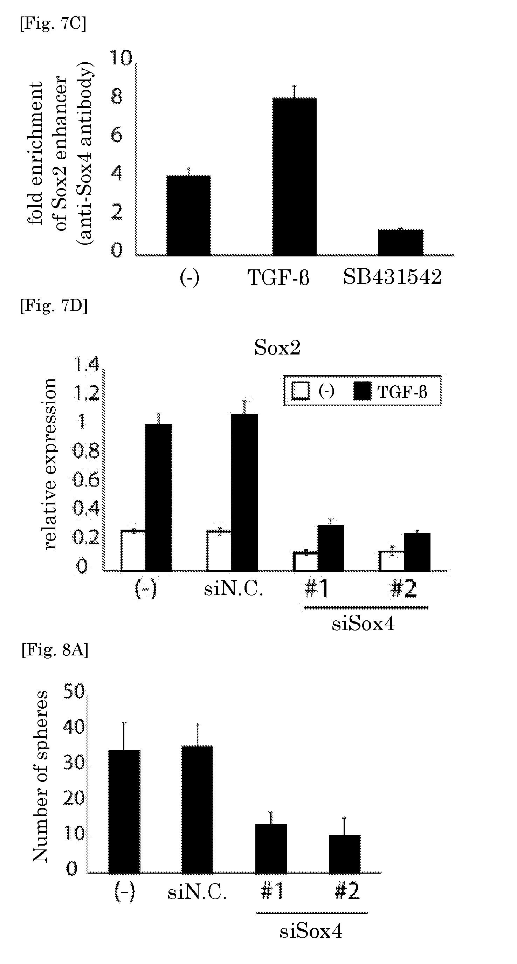

(Fig. 7.) Fig. 7A is the result of measuring the Sox2 mRNA expression levels in cells that have expressed excessive Sox4. Fig. 7B is the result of examining the impact of Sox4 knockdown due to siRNA on Sox2 mRNA expression levels. Fig. 7C is the result of a chromatin immunoprecipitation assay to verify that sox4 binds to the Sox2 enhancer region. Fig. 7D is the result of examining the impact of Sox4 knockdown due to siRNA on Sox2 expression induction by TGF-β.

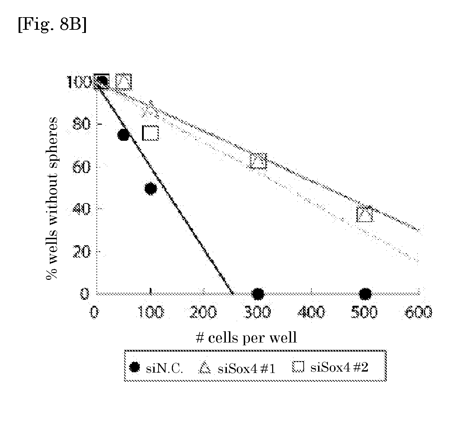

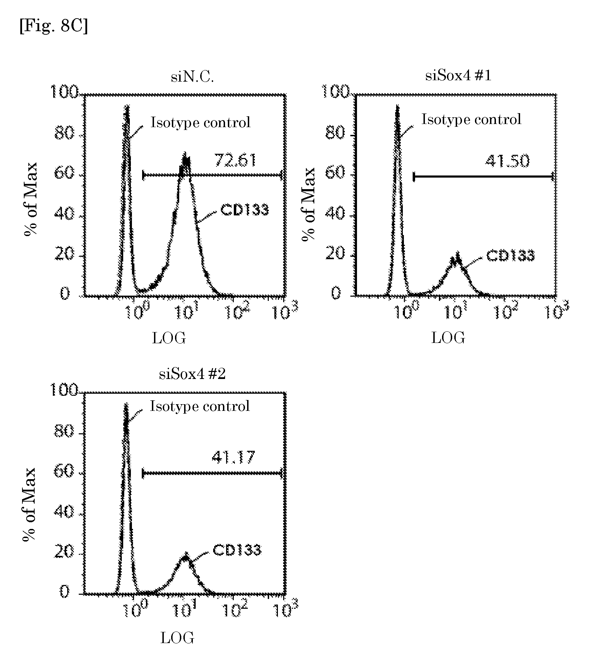

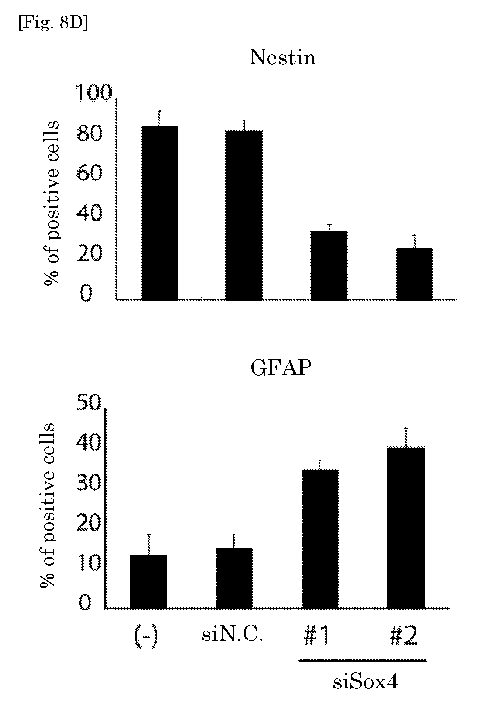

(Fig. 8.) Fig. 8A is the result of examining the impact of Sox4 knockdown due to siRNA on glioma stem cell sphere formation ability using sphere formation assay. Fig. 8B is the result of examining the impact of Sox4 knockdown due to siRNA on the glioma stem cell self-renewal ability using limiting dilution assay. Fig. 8C is the result of measuring the impact of Sox4 knockdown due to siRNA on the ratio of CD133-positive cells in the glioma spheres using flow cytometry. Fig. 8D is the result of measuring the percentage of Nestin-positive cells or GFAP-positive cells among all the cells to examine the impact of Sox4 knockdown due to siRNA on glioma stem cell differentiation.

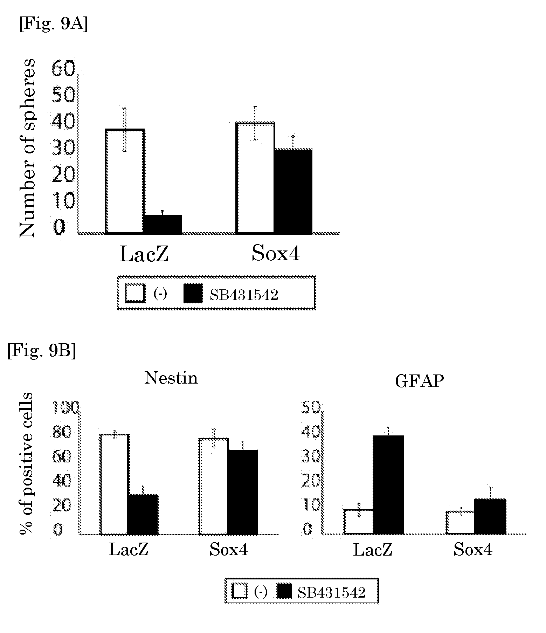

(Fig. 9.) Fig. 9A is the result of examining the impact of excessive Sox4 expression on stem cell maintenance by TGF-β inhibitors on glioma stem cells using sphere formation assay. Fig. 9B is the result of measuring the percentage of Nestin-positive cells or GFAP-positive cells among all the cells to examine the impact of excessive Sox4 expression on glioma stem cell differentiation.

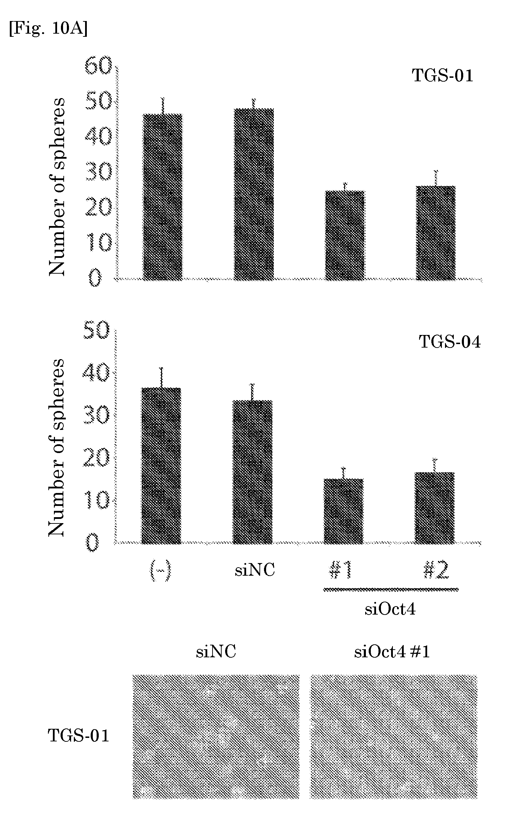

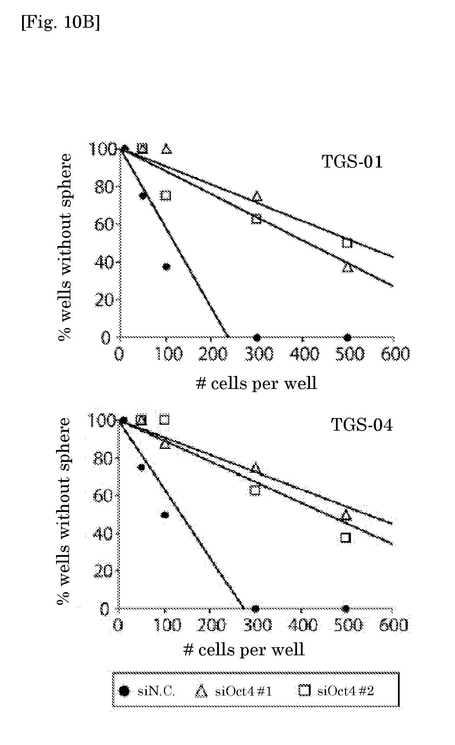

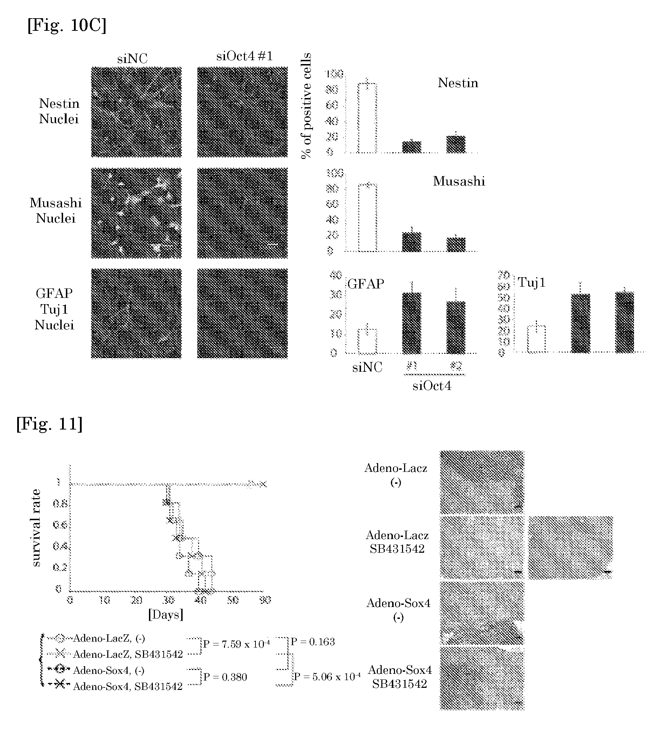

(Fig. 10.) Fig. 10A is the result of measuring the impact of Oct-4 knockdown due to siRNA on the sphere formation ability of glioma stem cells using sphere formation assay. Fig. 10B is the result of measuring the impact of Oct-4 knockdown due to siRNA on glioma stem cell self-renewal ability using limiting dilution assay. Fig. 10C is the result of examining the impact of Oct-4 knockdown due to siRNA on the percentages of Nestin-positive cells, Musashi-positive cells, and GFAP-positive cells among the total cells.
(Fig. 11.) Fig. 11 (Left) is the result of examining the survival period after transplanting glioma stem cells (Adeno-LacZ, SB431542) that had been preprocessed using a TGF-β inhibitor, and unprocessed glioma stem cells (Adeno-LacZ, (-)) into mice craniums. Transplants were also performed using glioma stem cells that expressed excessive Sox4 under the same conditions (Adeno-Sox4, SB431542 and Adeno-Sox4 (-)). The diagram on the right shows the results of histopathological examinations 30 days after transplant. The scale bar of the four photos on the left 50 µm (x20), and the scale bar of the photos on the right is 300 µm (x4).

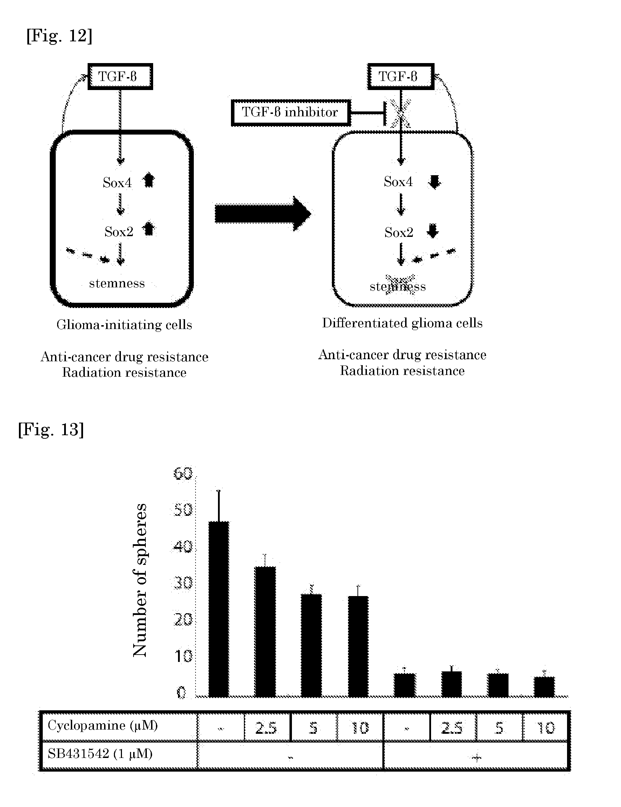

(Fig. 12.) Fig. 12 is a concept diagram to explain the mechanism of glioma stem cell cellularity maintenance by TGF-β signals. TGF-β directly induces Sox4 expression, and next, Sox4 promotes Sox2 expression, which maintains the glioma stem cell cellularity (left). TGF-β inhibitors inhibit the TGF-β-Sox4-Sox2 pathway, thus promoting glioma stem cell differentiation, and reducing tumorigenicity (right). The dotted line arrows show the possibility of other signal pathway involvement.
(Fig. 13.) Fig. 13 is the result of a sphere formation assay implemented to examine the impact of the Hedgehog-Gil1 pathway inhibitor cyclopamine on glioma stem cell differentiation.

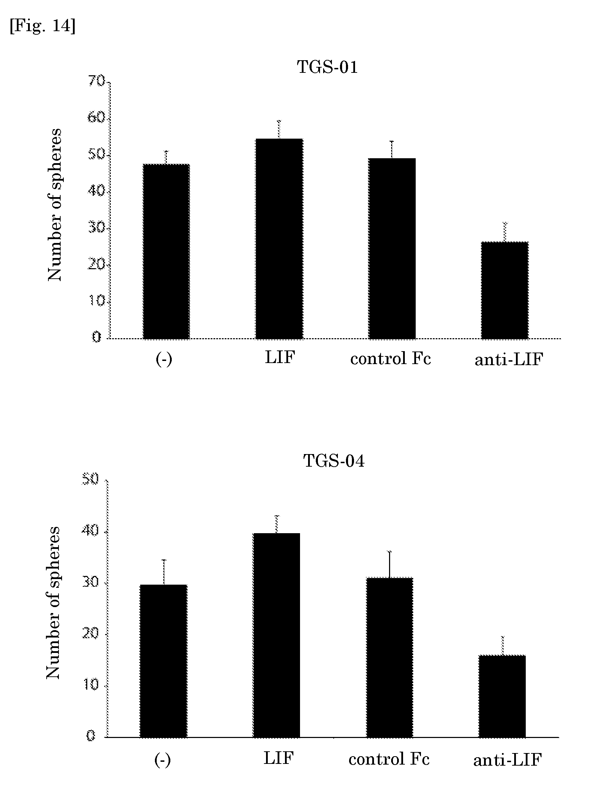

(Fig. 14.) Fig. 14 is the result of a sphere formation assay implemented to examine the role of leukemia inhibiting factor (LIF) on glioma stem cell differentiation.

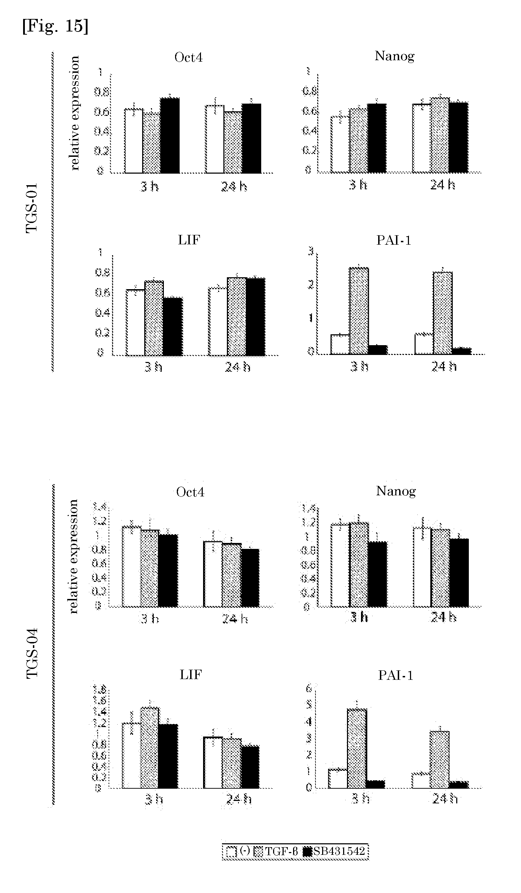

(Fig. 15.) Fig. 15 is the result of examining the impact of TGF-β stimulation on the expression of stem cell markers (Oct-4, NANOG, and LIF) in glioma stem cells. Plasminogen activator inhibitor (PAI-1) was measured as a positive control.

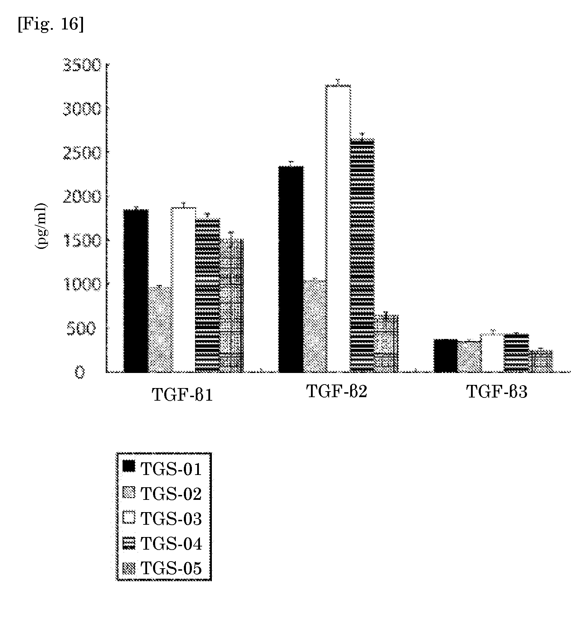

(Fig. 16.) Fig. 16 is the result of measuring the expression of TGF-β subtypes in cells TGS-01 to TGS-05 using ELISA.

### Format for Implementing the Invention

### Brain Tumor Stem Cell Differentiation Promoters

The brain tumor stem cell differentiation promoters for this invention include substances that inhibit the TGF-β-Sox4-Sox2 pathway.
In this detailed description, "brain tumor" means a tumor occurring in the intracranial tissue. Brain tumors are broadly classified using the WHO brain tumor tissue classification into neuroepithelial tissue tumors (gliomas, ependymomas, neurocellular tumors, fetal neuroectodermal tumors, etc.), nerve sheath tumors (neurilemomas, neurofibromas, etc.) meningeal tumors (meningiomas and other mesenchymal tumors, etc.), lymphatic tumors and hematopoietic neoplasms, germ cell tumors (germinomas, yolk sac tumors, teratomas, etc.), sellar tumors, and metastatic tumors.

In this detailed description, "brain tumor stem cell" is a cancer stem cell in a brain tumor. Brain tumor stem cells are known to express CD133, which is used as a marker.

Upon differentiation, brain tumor stem cells lose their tumorigenicity, and become sensitive to anti-cancer drugs and radiation. Consequently, promoting brain tumor stem cell differentiation improves the effects of anti-cancer drug treatments and radiotherapy, and can not only suppress growth of the brain tumor itself, but also suppress the risks of recurrence and metastasis.
The "brain tumor stem cell differentiation promoter" concerned in this invention promotes differentiation by working directly on the brain tumor stem cells. Cells differentiated from brain tumor stem cells (hereinafter sometimes called "brain tumor differentiated cells") lose their typical cancer stem cell characteristics, such as tumor formation ability, asymmetric cell division ability, and drug resistance ability.

As described above, there have been many reports to date regarding glioma brain tumor stem cells (i.e., glioma stem cells). The brain tumor stem cell differentiation promoter in this invention is naturally also used as a glioma stem cell differentiation promoter. "Glioma stem cells" are cancer stem cells in glioma with characteristics such as the expression of nerve stem cell antigens (stem cell markers; e.g., Nestin and Musashi, etc.), the formation of non-adhesive spherical cell masses called neurospheres or glioma spheres when cultivated in a serum-free culture in the presence of EGF and bFGF, and possessing self-renewal abilities. Consequently, glioma stem cells can be assayed by verifying the presence of at least one of these characteristics. While on the one hand glioma stem cells have strong tumorigenicity, and also have infiltration and metastatic abilities, on the other hand, they are known to possess resistance to anti-cancer drugs and radiotherapy.

Cells differentiated from glioma stem cells (hereinafter also called "differentiated glioma cells") are characterized by becoming adhesive cells without forming spheres even if cultivated in a serum-free culture in the presence of EGF and bFGF, not having self-renewal abilities, and expressing differentiated cell markers (e.g., GFAP and Tujl, etc.) without expressing stem cell markers. Consequently, glioma stem cells can be judged to have differentiated by verifying at least one of the following with the glioma stem cells: reduced sphere formation ability under designated conditions, reduced self-renewal abilities, reduced stem cell marker expression, and elevated differentiated cell marker expression. Further, sphere formation can be evaluated, for example, by culturing cells in a suitable medium, and then counting the number of spheres that form. In addition, self-renewal abilities can be evaluated by applying limiting dilution assay, for example. The expression of stem cell markers or differentiated cell markers can be measured according to well-known methods (for example, northern blotting, RNase protection assay, or RT-PCR, etc.)

Further, in this detailed description, "glioma" is the general name for tumors occurring from glioma cells, and include astrocytic tumors, oligodendrocytic tumors, ependymal cell tumors, and choroidal tumors, etc. Astrocytic tumors are classified into hairy cell astrocytomas (Grade I), subependymal giant cell astrocytomas (Grade I), pleomorphic xanthoastrocytomas (Grade II), diffuse astrocytomas (Grade II), anaplastic astrocytomas (Grade III), and glioblastomas (Grade IV). Oligodendrocytic tumors are classified into oligodendrogliomas (Grade II) and anaplastic oligodendrogliomas (Grade III), ependymal cell tumors are classified into ependymomas (Grade II) and anaplastic ependymomas (Grade III), and choroidal tumors are classified into choroid plexus papillomas (Grade I) and choroid plexus carcinomas (Grade III).

In this detailed description, the "TGF-β-Sox4-Sox2 pathway" is the name of the pathway in which TGF-β induces Sox4 expression in brain tumor stem cells, and the Sox4 then promotes Sox2. The inventors discovered that this pathway is necessary to maintain the stem cellularity of brain tumor stem cells.
In this detailed description, "substances that inhibit the TGF-β-Sox4-Sox2 pathway" are not particularly limited provided they are substances that reduce or eliminate the functions of the pathway in maintaining the stem cellularity of the brain tumor stem cells, and may indicate other substances that are involved with the pathway either directly or indirectly if the substance operates on either TGF--β, Sox4, or Sox2. Substances that can be cited as being indirectly involved with the pathway are, for example, SMAD2, SMAD3, Oct-4, and SMAD4, but are not limited to these.
As shown by the implementation examples described below, inhibiting the TGF-β-Sox4-Sox2 pathway promotes brain tumor stem cell differentiation, which reduces the tumorigenicity remarkably.

Ideally, the TGF-β-Sox4-Sox2 pathway inhibitor in this invention should be an expression inhibitor or function inhibitor for at least one selected from the group Sox2, Sox4, TGF-β, SMAD2, SMAD3, and Oct-4. as described in the implementation examples, inhibiting either Sox2, Sox4, TGF-β, SMAD2, SMAD3, or Oct-4 causes the stem cellularity of the brain tumor stem cells to be lost, and promotes differentiation. Further, in this detailed description, "expression" can include inhibition at either the transcription level or the translation level when expression is inhibited using the concepts included in transcription and translation. In addition, in this detailed description, "function" includes any of the functions of genes, transcription products, and proteins, and inhibiting functions means to inhibit all or some of any of these functions.

### Sox2 Inhibitors

Sox2 is known as a self-renewal gene in the same way as Oct-4 and NANOG, and plays an important role in maintaining the stem cellularity of embryonic stem cells. Further, it has been reported that when human or mouse fibroblasts are introduced together with Oct-4, KLF4, and c-Myc, induced pluripotent stem cells (iPS cells) are induced. (Takahashi and Yamanaka, 2006; Takahashi et al., 2007.) To date, it has also been reported that compared to non-malignant tissue, the expression of mRNA-level Sox2 is elevated in brain tumor tissues such as glioma tissue, but the way in which Sox2 functions in the growth of brain tumors is unclear. These inventors verified that the induction of Sox2 expression by TGF-β is necessary to maintain stem cellularity in brain tumor stem cells, and that brain tumor stem cell differentiation is induced by inhibiting Sox2 functions or expression.

Any substance can be used as a Sox2 expression inhibitor or function inhibitor, such as low-molecular compounds, high-molecular compounds, nucleic acids, peptides, or proteins, etc., and are not particularly restricted provided they are a substance that can suppress Sox2 functions, which maintain the stem cellularity of brain tumor stem cells. Specifically, anti-Sox2 antibodies, which neutralize Sox2 activation, and nucleic acids, which suppress Sox2 expression, can be cited, but the substances are not restricted to these.

In this detailed description, "antibody" includes antibody fragments, and the anti-Sox2 antibodies in this invention can be monoclonal antibodies, polyclonal antibodies, recombinant antibodies, human antibodies, humanized antibodies, chimera antibodies, single chain antibodies, Fab fragments, F(ab')2 antibodies, scFv, bispecific antibodies, or synthetic antibodies. These antibodies can be created using the methods published by the relevant commercial enterprises. For example, monoclonal antibodies can be obtained by isolating antibody-producing cells from non-human mammals immunized using Sox2, and fusing them with myeloma cells to create a hybridoma, and then refining the antibodies produced by the hybridoma. Further, polyclonal antibodies can be obtained from animal serum immunized using Sox2.
Once non-human monoclonal antibodies in which Sox2 activation has been neutralized have been obtained, the amino acid sequence can be specified to produce the antibodies using DNA recombination techniques. In addition, humanized antibodies and human antibodies should ideally be created using publically-known methods or their equivalent.
If the anti-Sox2 antibodies in this invention are low-molecular antibodies such as Fab fragments, F(ab')2 antibodies, or scFv, etc., they can be expressed by gene recombination using nucleic acids that code for low-molecular antibodies and, further, created by processing antibodies using papain or pepsin, etc.

In addition, double-stranded nucleic acids, antisense nucleic acids, and ribozymes, etc., which have RNAi effects, can be cited as "nucleic acids that suppress Sox2 expression". The RNAi effect is a mechanism that suppresses gene expression in specific sequences induced by double-stranded nucleic acids. Marker specification is extremely high, and as the method uses a gene expression suppression method that originally existed *in vivo,* it is highly safe.
siRNA can be cited, for example, as a double-stranded nucleic acid with RNAi effects. siRNA is double-stranded RNA with normally 19 to 30 bases, and ideally 21 to 25 bases, when used in mammalian cells. The Sox2 expression inhibitor in this invention, however, may have longer double-stranded RNA than what can be obtained as siRNA by cutting using enzymes (Dicer). Generally, double-stranded nucleic acids with RNAi effects have on the one hand base sequences that complement part of the marker nucleic acids, and on the other have the corresponding complementary sequences. Double-stranded nucleic acids with RNAi effects generally have two mutually-protruding bases at the 3' terminus ("overhang"), but blunt-ended types without any overhang are also acceptable. For example, a 25-base blunt-end RNA minimizes the activation of the interferon response genes, thus preventing off-target effects from arising from the sense chain, and has the advantage that its stability in serum is extremely high, and is thus suitable for use *in vivo.*
Double-stranded nucleic acids with RNAi effects can be designed using publically-known methods according to the base sequence of the marker DNA. Further, double-stranded nucleic acids with RNAi effects may also be double-stranded RNA, DNA/RNA chimera double-stranded nucleic acids, artificial nucleic acids, or any nucleic acid that has been embellished.
The following can be cited as examples of double-stranded nucleic acids with RNAi effects against Sox2: RNA comprising the base sequences described in Sequence Number: 1 and RNA with complementary base sequences, and RNA comprising the base sequences described in Sequence Number: 2 and RNA with complementary base sequences.

Antisense nucleic acids have base sequences that complement the marker genes (basically, mRNA, which is a transcription product), and generally are single-stranded nucleic acids ten to 100 bases long, or ideally 15 to 30 bases long. Gene expression is inhibited by introducing the antisense nucleic acid into the cell to cause hybridization with the marker gene. Antisense nucleic acids do not have to be completely complementary to the marker gene provided the effects that inhibit marker gene expression can be obtained. Antisense nucleic acids can be suitably designed by companies using publically-available software. Antisense nucleic acids may be either DNA, RNA, or a DNA/RNA chimera, or may have been embellished.

Ribozymes are nucleic acid molecules that hydrolyze marker DNA catalytically, and are configured from the antisense region, which has the marker RNA and its complementary sequence, and the central catalyst region, which is responsible for the cutting reaction. Ribozymes can be designed by companies using publically-available software. Ribozymes are generally RNA molecules, but DNA/RNA chimera molecules may be used.

Further, the double-stranded nucleic acids with RNAi effects, antisense nucleic acids, and any nucleic acids that code for ribozymes as described above can also be used as Sox2 expression inhibitors in this invention. Introducing vectors that include hanging DNA into the cell will express the double-stranded nucleic acids with RNAi effects, antisense nucleic acids, and ribozymes, and each of these manifest the effect of suppressing Sox2 expression.
DNA that codes for either of the double stands may also be used as a nucleic acid that codes for double-stranded nucleic acids with RNAi effects, and DNA that codes for one-stranded nucleic acids that can bind via a double-stranded nucleic acid loop may also be used. In the latter case, single-stranded RNA obtained from transcription within the cell hybridizes with its complement within the molecule to obtain a hairpin configuration. This RNA is called shRNA (short hairpin RNA). shRNA is cut by the loop using an enzyme (Dicer) when migrating to the cytoplasm, where it becomes double-stranded RNA and manifests RNAi effects.

### Sox4 Inhibitors

Sox2 is known to be an important molecule in the stem cellularity of nerve stem cells, but there are as yet no reports on the Sox4 functions regarding stem cellularity. There are reports that compared to normal brain tissue, Sox4 is over-expressed in brain tumor tissue such as glioma tissue, etc., but the role that it plays in brain tumor growth is unclear.
The inventors bound Sox4 to the Sox2 enhancer region to induce expression, and verified that it plays an important role in maintaining the tumorigenicity of the brain tumor stem cells.

Any Sox4 expression inhibitors or function inhibitors can be used, such as low-molecular compounds, high-molecular compounds, nucleic acids, peptides, and proteins, etc., and are not particularly limited provided they are substances that can inhibit the Sox4 function of inducing Sox2 expression. Specifically, anti-Sox4 antigens, which neutralize Sox4 activation, and nucleic acids that inhibit Sox4 expression can be cited, but are not limited to these.
Anti-Sox4 antibodies and nucleic acids that inhibit Sox4 expression can be created and used in the same way as the anti-Sox2 antibodies and nucleic acids that inhibit Sox2 expression described above. Further, the following can be cited as examples of double-stranded nucleic acids with RNAi infects on Sox4: RNA comprising the base sequences described in Sequence Number: 3 and RNA with their complementary base sequences, and RNA comprising the base sequences described in Sequence Number: 4 and RNA with their complementary base sequences.

### TGF-β Inhibitors

TGF-β signals are known to have combined cancer-suppressing effects and cancer-promoting effects. In other words, in certain cells they inhibit cancer cell growth, and in other cells they promote their growth. Cancer treatments based on TGF-β have been examined before, but an approach that spans these contradictory actions is mired in difficulties.
The inventors verified that in brain tumor stem cells, TGF-β has functions that maintain stem cellularity. Consequently, the differentiation of brain tumor stem cells can be promoted by inhibiting the functions or expression of TGF-β.

Any TGF-β expression inhibitors or function inhibitors can be used, including low-molecular compounds, high-molecular compounds, nucleic acids, peptides, and proteins, etc., and are not particularly limited provided they are substances that can inhibit the TGF-β induction of Sox4 expression. Specifically, anti-TGF-β antibodies, which neutralize TGF-β activation, TGF-β receptor antagonists, which inhibit TGF-β binding to the TGF-β receptors, and nucleic acids that suppress TGF-β expression (antisense nucleic acids, double-stranded nucleic acids with RNAi effects, and ribozymes, etc.) can be cited, but are not limited to these.
Anti-TGF-β antibodies and nucleic acids that inhibit TGF-β expression can be manufactured and used in the same way as anti-Sox2 antibodies and nucleic acids that inhibit Sox2 expression. Further, there are three TGF-β subtypes from TGF-β1 to TGF-β3, and any of these transmit signals by binding to the TGF-βI and TGF-βII receptors, but as shown in the reference examples described below, the subtypes expressed depend on the patient. Consequently, when using a TGF-β receptor antagonist as a TGF-β expression inhibitor or function inhibitor, one that is effective on patients who express any of the subtypes is ideal.
Further, using a substance that targets the TGF-β receptors as a TGF-β functions inhibitor is also desirable, of which TGF-β receptor expression inhibitors (nucleic acids that inhibit the expression of TGF-β receptors), dominant-negative (dominant inhibition) TGF-β receptors, and function-inhibiting antibodies against TGF-β receptors can be cited as examples. Function-inhibiting antibodies against TGF-β receptors and nucleic acids that inhibit TGF-β receptor expression can be created and used in the same way as the anti-Sox2 antibodies and nucleic acids that inhibit Sox2 expression described above. Publically-known dominant-negative TGF-β receptors and their equivalent can be used.
SB431542 (Inman *et al.,* 2002), A-78-03 (Tojo *et al.,* 2005), and LY364947 (Sawyer *et al.,* 2003) can be cited as examples of the TGF-β expression inhibitors or function inhibitors that can be used ideally in differentiation promoters for brain tumor stem cells in this invention.

### SMAD2 and SMAD3 Inhibitors

SMAD2 and SMAD3 are R-SMADs that are activated and transmit signals within the cell by being bound to the serine-threonine kinase receptors by activin, which belongs to TGF-β or the TGF-β superfamily. R-SMAD forms a complex with SMAD4, which is a co-SMAD, and migrates inside the nucleus to regulate the expression of various marker genes. As shown in the implementation examples described below, the inventors verified that Sox2 expression was suppressed by inhibiting the expression of SMAD2 and SMAD3. Further, they verified that the SMAD complex binds directly to the Sox4 promoter due to TGF-β stimulation by means of chromatin immunoprecipitation assay using antibodies on SMAD2 and SMAD3. From the above, it can be said that SMAD2 and SMAD3 inhibition inhibits the TGF-β-Sox4-Sox2 pathway by suppressing the expression of Sox2 or Sox4.

Any substance such as low-molecular compounds, high-molecular compounds, nucleic acids, peptides, or proteins, etc., can be used as SMAD2 or SMAD3 expression inhibitors or function inhibitors and are not particularly restricted provided they are substances in which brain tumor stem cell differentiation is induced as a result of their administration. Specifically, anti-SMAD2 antibodies, which neutralize SMAD2 activation, or anti-SMAD3 antibodies, which neutralize SMAD3 activation, or nucleic acids that suppress SMAD2 or SMAD3 expression, can be cited, but are not limited to these.
Anti-SMAD2 and anti-SMAd3 antibodies, and nucleic acids that inhibit SMAD2 and SMAD3 expression, can be created and used in the same way as anti-Sox2 antibodies and nucleic acids that inhibit Sox2 expression as described above. Further, RNA comprising the base sequences described in Sequence Number: 5, and double-stranded RNA comprising RNA with its complementary base sequence can be cited as examples of double-stranded nucleic acids that have RNAi effects on SMAD2. In addition, RNA comprising the base sequences described in Sequence Number: 6, and double-stranded RNA comprising RNA with its complementary base sequence can be cited as examples of double-stranded nucleic acids that have RNAi effects on SMAD3.

### Oct-4 Inhibitors

Oct-4 is a transcription factor of the POU family, and is known to be one of the self-renewal genes that play an important role in maintaining the stem cellularity of embryonic stem cells, and can also be used in iPS cell induction. As described in the implementation examples below, the inventors verified that brain tumor stem cell differentiation was also induced by inhibiting Oct-4 expression.

Any substance such as low-molecular compounds, high-molecular compounds, nucleic acids, peptides, and proteins can be used as Oct-4 expression inhibitors or function inhibitors with no particular restrictions provided they are substances in which brain tumor stem cell differentiation is induced as a result of their administration. Specifically, anti-Oct-4 antibodies, which neutralize oct-4 activation, and nucleic acids that inhibit Oct-4 expression, can be cited, but are not limited to these.
Anti-Oct-4 antibodies and nucleic acids that inhibit Oct-4 expression can be created and used in the same way as the anti-Sox2 antibodies and nucleic acids that inhibit Sox2 expression described above. Further, RNA comprising the base sequences described in Sequence Number: 51, and double-stranded RNA comprising RNA with its complementary base sequence, and RNA comprising the base sequences described in Sequence Number: 52, and double-stranded RNA comprising RNA with its complementary base sequence, can be cited as double-stranded nucleic acids that have RNAi effects against Oct-4.

### Therapeutic Agents

The therapeutic agent for brain tumors in this invention includes a differentiation promoter for the brain tumor stem cells concerned with the invention as described above.
The therapeutic agent for brain tumors in this invention manufactures the agent in regular therapeutic agent format, which can be administered orally or parenterally, and systemically or locally. For example, IV injection such as drip, intramuscular injection, subcutaneous injection, suppositories, intestinal infusion, and oral enteric coated capsules can be selected, and a suitable method of administration chosen depending on the patient's age and symptoms.
In the case of the therapeutic agent for brain tumors in this invention, direct intracranial administration is desirable. For example, direct administration of the drug to the affected part of the brain is possible via a cannula that is inserted using stereotaxy. The method involved is desirable not only as it enables the therapeutic agent to be administered directly to the affected part, but also because it does not affect other tissue. The drug is perfused for a set period while the cannula is fixed in place.

In this detailed description, "regular therapeutic agent" is a suitably-mixed pharmaceutical using a vector that can be pharmaceutically tolerated. There is no particular restriction to the therapeutic agent format, which can be suitably selected and used according to the treatment objectives. Typical examples that can be cited include tablets, pills, powders, liquids, suspensions, emulsions, granules, capsules, suppositories, and injections (liquids, suspensions, and emulsions). These drugs should be prepared using the methods normally employed.

In this detailed description, "vector that can be tolerated pharmaceutically" means a substance that can be administered together with the differentiation promoter for the brain tumor stem cells in this invention. A vector that can be tolerated pharmaceutically should be tolerable pharmacologically and pharmaceutically, and has no particular restrictions. The following can be cited as examples, but are not limited to these: Organic solvents that are tolerated pharmacologically, such as water, saline, phosphate buffer, dextrose, glycerol, and ethanol, and collagen, polyvinyl alcohol, polyvinylpyrrolidone, carboxyvinyl polymers, carboxymethylcellulose sodium, sodium polyacrylate, sodium alginate, soluble dextran, carboxymethyl starch sodium, pectin, methylcellulose, ethylcellulose, xanthan gum, Arabian gum, casein, agar, polyethylene glycol, diglycerin, glycerin, propylene glycol, vaseline, paraffin, sterile alcohol, stearic acid, human serum albumin, mannitol, sorbitol, lactose, surfactants, excipients, flavorings, preservatives, stabilizers, buffers, suspensions, isotonics, bonding agents, disintegrators, lubricants, fluidic accelerators, and masking agents.

Further, if nucleic acid is included in the therapeutic agent of this invention, pharmaceuticals can be made by enclosing the nucleic acid in a carrier such as a ribozyme, high-molecular micelle, or cation carrier. In addition, a nucleic acid carrier such as protamine may also be used. Ideally, antibodies should be bound to these carriers to target the affected part. Moreover, it is also possible to improve retentivity by binding cholesterol to the nucleic acid. Furthermore, the pharmaceutical components of the invention include nucleic acids that code for siRNA, and if expressed within the cell after administration, the relevant nucleic acid can be inserted into a viral vector such as a retrovirus, adenovirus, or Sendai virus, or a non-viral vector such as a ribozyme, and administered into the cell.

Further, the amount of active ingredients included in the therapeutic agent in this invention can be adjusted depending on the type of active ingredients used by the manufacturer. For example, in the case of nucleic acids, the dose is 0.025 to 1000 mg/m² per day, and ideally is 0.1 to 500 mg/m², and more ideally can be set to 10 to 400 mg/m², but this is not restricted.
The therapeutic agent in this invention should ideally be administered long-term to work on the glioma stem cells. In the case of long-term administration, repeatedly alternating short periods of administration with periods of no administration is also acceptable and, for example, the periods of administration and periods of no administration can be one to ten days each. In addition, the therapeutic agent in this invention should ideally be used in combination with other anti-cancer drugs and methods of cancer treatment.

The therapeutic agent in this invention is effective on all brain tumors in which the stem cellularity of the brain tumor stem cells is maintained by the TGF-β-Sox4-Sox2 pathway.
For example, gliomas can be targeted, and the therapeutic agent used ideally even in treatments for anaplastic astrocytic cell tumors and glioblastomas, for which prognosis is poor and there have been no effective treatments to date. Further, medulloblastoma , which requires Sox2 for its tumorigenicity ability (Sutter, R, et al., Oncogene (2010) doi: 10.1038/onc. 2009.472) is also one of the brain tumors is which the therapeutic agent in this invention can be used favorably.

Further, in this detailed description, "treatment" describes the causation of at least one of the following: reduced tumor size (delayed or stopped), inhibition of tumor metastasis (delayed or stopped), inhibition of tumor growth (delayed or stopped), or the alleviation of single or multiple symptoms involving the brain tumor.

### Treatment Methods

In addition, this invention supplies methods of treating brain tumors, including processes for administering the therapeutic agents for the brain tumor in this invention and, if the expression level is elevated compared to normal tissue, a process for measuring the expression levels of Sox2 and/or Sox4 in the brain tumor tissue sampled from the patient.
The inventors showed that the TGF-β-Sox4-Sox2 pathway is indispensable in maintaining the stem cellularity of brain tumor stem cells, but it is undeniable that in living organisms, there exist other pathways that are also involved. For example, as described in the reference examples below, there are at least two in glioma stem cells: those dependent on the Hedgehog-Gil1 pathway, and those dependent on the TGF-β-Sox4-Sox2 pathway, and among those dependent on the TGF-β signal pathway, there are those that operate via LIF rather than Sox4-Sox2.
The therapeutic agents in this invention inhibit the TGF-β-Sox4-Sox2 pathway, and so the possibility of effectiveness is great when tissue is sampled from the affected part of the patient's brain tumor, and the expression levels of Sox2 and/or Sox4 are measured, and those expression levels are elevated.
Brain tumor tissue can, for example, be sampled from patients using a cannula through stereotaxy. Further, the expression of Sox4 and Sox 2 in the sampled tissue can be implemented using standard methods such as, for example, northern blotting, RNase protection assay, or RT-PCR, etc.

### Screening Methods

Moreover, this invention supplies a screening method to determine treatment methods for patients with brain tumors, including a process for measuring the levels of Sox2 and/or Sox4 expression in the brain tumor tissue sampled from the patient, and a process for comparing the relevant expression levels with the expression levels in normal tissue.
Depending on the method applied, it is possible to administer the therapeutic agents by specifying patients in whom there is a high possibility that the therapeutic agents in this invention are effective.

The publication of all patent and non-patent documents quoted in this detailed description have been incorporated with reference to the detailed description overall.

### Implementation Examples

Specific explanations based on implementation examples of the invention are described below, but the invention is in no way limited to these alone. Corporations can change the invention to various configurations without deviating from the significance of the invention, and the changes applied can also be incorporated into the scope of the invention.

The materials and methods using in the implementation examples are described below. Glioma Samples
In the following implementation examples, Grade IV glioma samples were taken during surgery from patients from whom consent was obtained following the receipt of approval from the clinical trials oversight committee of the University of Tokyo Hospital. (See Table 1.)

**Table 1**

| Sample No. | Age | Sex | Diagnosis | WHO Grading |
|---|---|---|---|---|
| TGS-01 | 54 | Male | GBM | IV |
| TGS-02 | 60 | Male | GBM | IV |
| TGS-03 | 69 | Female | GBM | IV |
| TGS-04 | 68 | Male | GBM | IV |
| TGS-05 | 58 | Male | GBM | IV |

### Immunostaining

Glioma-initiating cells (GICs) were seeded on glass slides that had been coded using poly-L-ornithine (Sigma) and fibronectin (Sigma), and were then cultured for seven days together with various ligands or inhibitors in serum-free culture. The cells were fixed using 3.7% paraformaldehyde, and then the cell membrane was made permeable using 0.3% Triton X-1000 including PBS, and incubated together with various antibodies.

### Flow Cytometry

The cells were separated into single cells, and marked using phycoerythrin marker CD133 antibodies. The expression levels were measured using EXPO32 ADC software through a Beckman C EPICS XL flow cytometer.

### Sphere Formation Assay

GICs were cultivated for seven days using a non-tissue-culture-treated-flask with vent cap (BD Bioscience). The floating spheres were counted for each sample within five fields of vision under a microscope.

### RNAi

The siRNA described in the table below were purchased from Invitrogen, and introduced to the cells according to the written explanation using an oligofectamine transfection agent (Invitrogen). siRNA is blunt-ended double-stranded RNA in which one strand is RNA comprising the base sequence described in the following table. Stealth siRNA 12935-400 was used as a negative control.

**Table 2**

| Marker Gene | siRNA | Base Sequence | Sequence No. |
|---|---|---|---|
| Sox2 | siSox2 No. 1 | AACCCAUGGAGCCAAGAGCCAUGCC | 1 |
| Sox2 | siSox2 No. 2 | UAGUGCUGGGACAUGUGAAGUCUGC | 2 |
| Sox4 | siSox4 No. 1 | UUUGCCCAGCCGCUUGGAGAUCUCG | 3 |
| Sox4 | siSox4 No. 2 | UUGUCGCUGUCUUUGAGCAGCUUCC | 4 |
| SMAD2 | siSMAD2 | AACAGCCUUUACAGCUUCUC | 5 |
| SMAD3 | siSMAD3 | CCAGAGAGUAGAGACACCAGUUCUA | 6 |
| Oct-4 | siOct-4 No. 1 | UCACCUUCCUCCAACCAGUUGCCC | 51 |
| Oct-4 | siOct-4 No. 2 | AUCUGCUGCAGUGUGGGUUUCGGGC | 52 |

### Adenoviruses

The total cDNA length of Sox2 and Sox4, which were labeled using FLAG-tags, were cloned on the pENTR vector (Invitrogen), and inserted into the adenovirus genome through recombination between the pENTR vector and pAd/CMV/V5-DEST vector using LR Clonase. After linealization using Pac1, the HEK293A cells were stained using pAd/CMV/Sox2 or pAd/CMV/Sox4. The virus particles were isolated using three freeze thawing cycles, and the HEK293A cells stained again for amplification.

### Cytolysis and Immunoblotting

The cells were dissolved in a buffer including 1% Nonidet P-40, 20 mM Tris-hydrochloride (pH7.4), 150 mM sodium chloride, 1 mM PMSF, 1% aprotinin, and 5 mM EDTA. The proteins refined from the cytolized product was supplied to SDS-PAGE, and transferred to a Fluoro Trans W Membrane (Pall). Immunoblotting was implemented using various types of antibodies.

### Quantitative Real-Time PCR

Quantitative real-time PCR was implemented according to the method by Ikushima et al. (EMBO J. 2008, 27: 2955-2965). All samples were implemented in triplicate. The data was normalized using glyceraldehyde 3-phosphate dehydrogenase (GAPDH). Further, the primers used in each expression analysis were as described below.

**Table 3**

| Marker Gene | Base Sequence | Sequence No. |
|---|---|---|
| GAPDH | F: GAAGGTGAAGGTCGGAGTC | 7 |
| | R: GAAGATGGTGATGGGATTTC | 8 |
| Sox2 | F: TGCGAGCGCTGCACAT | 9 |
| | R: TCATGAGCGTCTTGGTTTTCC | 10 |
| Sox4 | F: CTGCGCCTCAAGCACATG | 11 |
| | R: TTCTTCCTGGGCCGGTACT | 12 |
| Oct-4 | F: GGAGGAAGCTGACAACAATGAAA | 13 |
| | R: GGCCTGCACGAGGGTTT | 14 |
| NANOG | F: AGAACTCTCCAACATCCTGAACCT | 15 |
| | R: TGCCACCTCTTAGATTTCATTCTCT | 16 |
| LIF | F: TCTTGGCGGCAGGAGTTG | 17 |
| | R: CCGCCCATGTTTCCA | 18 |
| PAI-1 | F: GGCTGACTTCACGAGTCTTTCA | 19 |
| | R: ATGCGGCTGAGACTATGACA | 20 |
| p15^{INK4b} | F: GGTAATGAAGCTGAGCCCAGGT | 21 |
| | R: GATAATCCACCGTTGGCCGTA | 22 |
| p21^{WAF1} | F: GCGACTGTGATGCGCTAATG | 23 |
| | R: CCAGTGGTGTCTCGGTGACA | 24 |
| p27^{KIP1} | F: GGACTTGGAGAAGCACTGCAGA | 25 |
| | R: TCCACCTCTTGCCACTCGTACT | 26 |
| c-Myc | F: CCACACATCAGCACAACTACGC | 27 |
| | R: CGGTTGTTGCTGATCTGTCTCA | 28 |
| TGF-β1 | F: GACTTCCGCAAGGACCTCGGC | 29 |
| | R: GCGCACGATCATGTTGGACAG | 30 |
| TGF-β2 | F: CCTGTCTACCTGCAGCACTCGA | 31 |
| | R: GGCGGCATGTCTATTTTGTAAACCTCC | 32 |
| TGF-β3 | F: TGGCTGTTGAGAAGAGAGTCCA | 33 |
| | R: CAAGTTGCGGAAGCAGTAATTG | 34 |
| TGF-β type II receptor | F: ATAAGGCCAAGCTGAAGCAG | 35 |
| | R: CTTCTGGAGCCATGTATCTTG | 36 |
| ALK5 | F: TCGCCCTTTTATTTCAGAGGGTACT | 37 |
| | R: ACAGCAAGTTCCATTCTTCTTTACC | 38 |
| SMAD2 | F: CCCATCGAAAAGGATTGCCACA | 39 |
| | R: TGCATGGAAGGTTTCTCCAACC | 40 |
| SMAD3 | F: GGACGACTACAGCCATTCCA | 41 |
| | R: TTCCGATGTGTCTCCGTGTCA | 42 |
| SMAD4 | F: CTTTGAAATGGATGTTCAG | 43 |
| | R: CATCCTGATAAGGTTAAGGG | 44 |

### Limiting Dilution Assay

The sphere cells were separated into single cells, and placed in 200 µL of serum-free culture on a 96-hole plate. After seven days of culturing, the percentage of wells that did not include spheres was calculated against each cell-plating density, and plotted against the number of cells for each well.

### Chromatin Immunoprecipitation

Chromatin immunoprecipitation was implemented using the method described by Koinuma et al. (Molecular and Cellular Biology, 2009, 29:172-186, 10.1128/MCB, 01038-08). After reverse cross-linking, the DNA was processed using proteinase K, and refined using a PCR purification kit (Qiagen). The DNA was eluted into 30 µL of TE, and used in either PCR or quantitative real-time PCR. The primers used in the PCR are as described below.

**Table 4**

| Marker Gene | Base Sequence | Sequence No. |
|---|---|---|
| HPRT1 | F: TGTTTGGGCTATTTACTAGTTG | 45 |
| | R: ATAAAATGACTTAAGCCCAGAG | 46 |
| Sox4 | F: CCTCTTGAACAACATGCAGCTTT | 47 |
| | R: TGATGTATTGTAAGCCCTCAATGAA | 48 |
| Sox2 | F: GGATAACATTGTACTGGGAAGGGACA | 49 |
| | R: CAAAGTTTCTTTTATTCGTATGTGTGAGCA | 50 |

### Intracranial Proliferation Assay

Glioma-initiating cells (living cells 5 x 104 in 5 µL of DMEM/F 12 culture) were injected stereotaxically at a depth of 3mm into the right cerebral hemisphere of five-week-old female BALB/c nu/nu mice. All animal testing was implemented according to the policies of the University of Tokyo animal testing committee.

### Statistical Analysis of Microarray Data

The microarray data was acquired from GEO (http://www.ncbi.nim.nih.gov/geo/) and ArrayExpress (http://www.ebi.ac.uk/microarray/). Statistical software R was used in the data analysis.

### Implementation Example 1: Regulation of Glioma Sphere and Differentiated Glioma Cells, and Evaluation of their Characteristics

To clarify the mechanism by which glioma stem cellularity is maintained, TGS-01 and TGS-04 were first cultured in serum-free culture as described above to obtain spheres. (See Fig. 1A Left.) Both had self-renewal abilities, and showed similar action to that of the original cancer when transplanted into the brains of immunosuppressed mice. On the other hand, when the same samples were cultivated in 10% fetal bovine serum, the spheres did not form. (See Fig. 1A Right.) Cells cultivated in this way are sometimes called "differentiated glioma cells" or "differentiated cells" below.

CD133 (Prominin-1) has been reported as a glioma-initiating cell marker. (See non-patent document 1.) Here, the cells were separated into single cells, and the CD133 expression levels measured using flow cytometry. The results are shown in Fig. 1B. An increase in the percentage of CD133-positive cells in the glioma spheres was verified by comparison with the differentiated glioma cells. (Spheres 72.0%, differentiated cells 1.6%.) Further, the spheres were able to subculture continuously, and so the manifestation of Nestin (a neural precursor cell marker) and the fact that the cells were able to clone and themselves and self-renew were verified. (See Fig. 1C.)
In addition, an increased in glioma-initiating cells was verified in the TGS-01 and TGS-04 spheres using intracranial proliferation assay. (Not shown in the data.)

### Implementation 2: Effects of TGF-β Signal Inhibition against the Tumorigenicity of Glioma-Initiating Cells

To clarify the role of TGF-β signals in glioma-initiating cells, first the effects due to TGF-β signal inhibition were examined. A sphere formation ability assay was implemented in the presence of either TGF-β or a TGF-β inhibitor (SB431542, 1 µM). SB431542 is a TGF-β type I receptor (ALK5) kinase inhibitor (Inman et al., Molecular Pharmacology, 2002, 62: 65-74). The results are shown in Fig. 2A and Fig. 2B. When processed using SB431542, the sphere formation ability of glioma-initiating cells was conspicuously reduced.

SB431542 can inhibit not only the TGF-β type I receptor (all5), but also activin/nodal type I receptor signals, so to identify the role of TGF-β signals, the effects of a TGF-β receptor II/Fc chimera on glioma-initiating cells were examined. The glioma-initiating cells were separated into single cells, and a sphere assay implemented in the presence of either human TGF-βRII/Fc chimera (1 µg/mL), or IgG1Fc (1 µg/mL) as a control. The results are shown in Fig. 2C. The efficiency of glioma-initiating cells processed using TGF-βRII/Fc in forming spheres was low.

Similar sphere formation assay effects were obtained even with processing using A-78-03, which is another TGF-β signal inhibitor (Tojo et al., Cancer Science, 2005, 96: 791-800) or LY364947 (Sawyer et al., Journal of Medicinal Chemistry, 2003, 46: 3953-3956), or using adenovirus staining with SMAD7 cDNA, which is an intrinsically negative regulatory factor in TGF-β signals. (See Fig. 2E.) Further, in the presence of SB431542, the spherical growth patterns of spheres that had once formed were lost, and they became adhesion cells. (See Fig. 2F.) The reduced number of sphere formations in glioma-initiating cells dye to TGF-β signal inhibition hints that the self-renewal ability was lost.

Next, the effects of TGF-β (100 pM) or SB431542 (1 µM) were examined using flow cytometry. In the presence of TGF-β inhibitors, the percentage of CD133-positive cells in the glioma spheres was reduced. (See Fig. 3A.) Further, to examine the expression of neural precursor cell markers or neural cell differentiation markers in the glioma spheres, the spheres (TGS-01) were separated into single serum-free culture, and seeded on glass slides that had been coded using poly-L-ornithine (Sigma) and fibronectin (Sigma), and were then cultured for seven days together with TGF-β (100 pM) or the inhibitor SB431542 (1 µM). The cells were fixed using 3.7% paraformaldehyde, and the cell membrane was made permeable using 0.3% Triton X-1000 including PBS, and incubated together with antibodies before immunostaining was implemented.
TGF-β inhibition reduced the Nestin and Musashi (neural precursor cell marker)-positive cells, and increased either the GFAP (astrocyte differentiation marker) or Tuj 1 (βIII-tubulin, neural cell marker).

The results described above hinted that TGF-β signals maintain the tumorigenicity and stem cellularity of glioma-initiating cells. Conversely, even adding TGF-β did not greatly influence glioma-initiating cell sphere formation ability, CD133-positive ratio, or marker expression. (See Fig. 2A, Fig. 3A, and Fig. 3B.) This is thought to be because glioma-initiating cells express all the main components of the TGF-β signal and due to the production of sufficient secretory TGF-β signals to maintain stem cellularity by secreting TGF-β1 and TGF-β2.

### Implementation Example 3: Maintenance of Stem Cellularity in Glioma- Initiating Cells by Sox2, and the Induction of Sox2 Expression due to TGF-β

To clarify the mechanism by which the stem cellularity of glioma-initiating cells is maintained by TGF-β signals, the effects of TGF-β and 31542 on the expression of stem cell markers were examined.
The results are shown in Fig. 4A. The expression of Sox2 mRNA, which is a HMG-box factor, was induced 24 hours after processing using TGF-β (100 pM), but when using SB431542 (1 µg), Sox2 expression was suppressed after 24 hours, and the low expression level continued subsequently for seven days.
In contrast, the expression of other pluripotent stem cell-related molecules such as Oct-4, NANOG, and LIF, etc., were not greatly affected by processing using TGF-β or an inhibitor. (See Fig. 14.) Further, it has been reported that NANOG and LIF are induced in many cells by stimulating TGF-β. (Xu et al., Cell Stem Cell, 2008, 3: 206; Bruna et al., Cancer Cell, 2007, 11: 147-160.)

In addition, siRNA (siSMAD2 and siSMAD3) were transfected to SMAD2 and SMAD3 in TGS-01 cells, and incubated for 24 hours, and then processed for TGF-β (100 pM), and the expression levels measured. The measured values were normalized using GAPDH mRNA, and the results are shown in Fig. 4B. Sox2 induction using TGF-β was clearly suppressed in the presence of siRNA on SMAD2 and SMAD3. (See Fig. 4B). This fact shows that Sox2 expression is suppressed by TGF-β-SMAD signals. Moreover, it was also verified that the 24-hour processing using TGF-β (100 pM) or SB431542 (1 µM) suppressed the expression of Sox2 protein levels. (See Fig. 4C.) In the diagram, a-tubulin is the loading control.

Further, to examine the effects of Sox2 expression knockdown due to siRNA on the glioma sphere forming abilities and self-renewal abilities, the TGS-01 cells were separated into single cells, and either a control (NC) or siRNA against Sox2 (siSox2 No. 1 or siSox2 No. 2) were introduced and cultured for seven days, and then either a sphere formation assay or limiting dilution assay were implemented. The results of the control are shown in are shown in Fig. 4D, the results of the siRNA against Sox2 (siSox2 No. 1) are shown in Fig. 4E, and he results of the siRNA against Sox2 (siSox2 No. 2) are shown in Fig. 4G respectively. The sphere formation abilities and self-renewal abilities of the glioma-initiating cells were conspicuously reduced by the Sox2 knockdown.
In addition, the effects of the Sox2 knockdown on the percentage of CD133-positive cells among the TGS-01 cells were measured using flow cytometry. The results are shown in Fig. 4F. The Sox2 knockdown reduced the CD133-positive cells (from 75.1% to either 29.3% or 35.9%).
Moreover, to examine the effects of Sox2 knockdown using siRNA on glioma-initiating cell differentiation, the percentages of Nestin-positive cells and GFAP-positive cells among all the cells were measured. When Sox2 was knocked down, the percentage of Nestin-positive cells was reduced, and the percentage of GFAP-positive cells increased (see Fig. 4H), and thus the promotion of differentiation was verified.

From the above, it can be understood that Sox2 is indispensable for the maintenance of stem cellularity in glioma-initiating cells. Further, the down-regulation of Sox2 manifestation after 24 hours of SB431542 processing is hinted not by the results, which stole the stem cellularity of the glioma-initiating cells, but rather the causes.

### Implementation Example 4: Effects of TGF-β Inhibitors on Cells that Over-Express Sox2

To investigate more deeply the role of Sox2 in maintaining stem cell cellularity due to TGF-β, adenovirus coated with Sox2 cDNA was stained, and the effects of TGF-β inhibition on glioma-initiating cells that had been made to over-express Sox2 was examined.
Even if SB431542 was administered to glioma-initiating cells that had been made to over-express Sox2, sphere formation abilities were reduced only slightly compared to cells that had been made to over-express LacZ. (See Fig. 5A.) Further, even if SB431542 was administered to cells that over-expressed Sox2, from the fact that the percentage of Nestin-positive cells did not fall, and the percentage of GFAP-positive cells did not increase (see Fig. 5A), it was determined that glioma cells maintain stem cellularity.
From these results, it was understood that loss of stem cellularity using TGF-β inhibitors was due to the down-regulation of Sox2, which maintains the stem cellularity of glioma-initiating cells.

### Implementation Example 5: The Role of Sox4 in Glioma-Initiating

The induction of Sox2 expression by TGF-β was observed 24 hours after stimulation, but not observed after three hours. (See Fig. 4A.) Further, the effects of TGF-β on Sox2 expression in the presence of cycloheximide, which is a protein synthesis inhibitor, were examined. TGF-β (100 pM) processing was implemented for 24 hours, and after processing the TGF-β for 30 minutes, the cycloheximide processing was started, and the Sox2 expression levels were measured. The measured values were normalized using GAPDH mRNA, and the results are shown in Fig. 6A. In the presence of cycloheximide, which is a protein synthesis inhibitor, the induction of Sox2 expression was reduced. From these facts, it is thought that Sox2 expression is not induced directly by TGF-β, but rather, that it is controlled by other factors that are induced by TGF-β.
Here, to examine candidate genes that mediate Sox2 expression induction using TGF-β, candidate genes were selected from the published microarray data (Beier *et al.,* 2007; Bruna *et al.,* 2007; Guenther *et al.,* 2008; Lee *et al.,* 2006; Tso *et al.,* 2006) using the following criteria.
(1) Genes whose expression in glioma-initiating cells was elevated compared to tumor cells
(2) Genes induced directly by TGF-β in glioma cells, and suppressed by TGF-β inhibitors
(3) Genes with expression levels in glioma cells correlated to the expression levels of Sox2
As a result, transcription factor Sox4 was identified from among the genes with high expression in glioma-initiating cells as being a TGF-β marker gene.

Further, when Sox4 mRNA expression levels in the TGS-01 and TGS-04 cells (spheres), and in the differentiated glioma cells, were measured, it was verified that the expression levels in the spheres had risen. (See Fig. 6B.) In the diagram, a-tubulin is a loading control.
In addition, to verify whether or not Sox4 expression is regulated by the TGF-β signals, the amount of sox4 mRNA was measured after processing for three hours and 24 hours using TGF-β (100 pM) or SB431542 (1 µM) (see Fig. 6C), and the amount of Sox4 proteins was measured 24 hours after processing (see Fig. 6D.) Sox4 mRNA expression in TGS-01 and TGS-04 cells was induced immediately after TGF-β stimulation (i.e., after three hours) and, conversely, down-regulated by the TGF-β inhibitors.
To verify that Sox4 is a direct TGF-β marker, chromatin immunoprecipitation assay was implemented using antibodies to SMAD2 and SMAD3, which are DNA binding signal mediators for the TGF-β signals. Specifically, TGS-01 cells were processed for 1.5 hours using either TGF-β (100 pM), or SB431542 (1 µM), and the eluted DNA was supplied for either real-time PCR or RT-PCR. In real-time PCR, the measured values were normalized using the first introns of hypoxanthine phosphoribosyltransferase (HPRT). The results are shown in Fig. 6E. SMAD complexes were bound directly to Sox4 promoters by TGF-β stimulation, and this binding was clearly controlled by SB431542. Further, Sox4 induction by TGF-β was not influenced by cycloheximide. (See Fig. 6F.)

From the above results, it was understood that sox4 is a direct TGF-β signal marker gene.

### Implementation Example 6: Promotion of Sox2 Expression Using Sox4 Binding to the Sox2 Enhancer Region, and the Relevant Bonds

The effect of the over-expression of Sox4 on Sox2 expression was examined. Adenovirus-Sox4 or LacZ were stained on TGS-01 cells, and then recycled after 24 hours, and their respective expression amounts measured. The measured values were normalized using GAPDH mRNA. The results are shown in Fig. 7A. The over-expression of Sox4 in glioma-initiating cells up-regulated Sox2 expression.

Further, the effects of Sox4 knockdown due to siRNA on Sox2 expression was examined. The results are shown in Fig. 7B. The top shows the expression levels of Sox2 and Sox4 mRNA, and the bottom shows the expression level of Sox4 proteins.

### Sox2 expression was controlled by Sox4 knockdown. (See Fig. 7B.)

Meanwhile, Sox2 mRNA expression under the control of a cytomegalovirus (CMV) promoter was down-controlled by sox4 knockdown due to siRNA. This fact shows that Sox2 mRNA is not a direct siRNA marker for Sox4.
From the above, it was understood that Sox2 expression is up-controlled at the transcription level by Sox4.

To verify that this control is direct, chromatin immunoprecipitation assay was implemented using Sox4 antibodies. It has previously been reported that the enhancer region located in the Sox2 gen 3' flanking region is important for controlling Sox2 expression. (Chew et al., Molecular and Cellular Biology,2005, 25:6031-6046; Tomioka et al., Nucleic Acids Research, 2002, 30: 3202-3213.) This region includes the consensus binding motif CATTGTA to Sox4. (Liao et al., Oncogene, 2008, 27: 5578-5589.)
Here, TGS-01 cells were processed for 24 hours using either TGF-β (100 pM), or SB431542 (1 µM), and the eluted DNA analyzed using quantitative real-time PCR. The measured values were normalized using HPRT first intron quantities. The results are shown in Fig. 7C. Sox4 recruitment to the sox2 enhancer region increased due to 24 hours of stimulation by TGF-β, and the recruitment was clearly reduced by SB431542. These results are thought to be based on Sox4 expression regulation by TGF-β or a TGF-β inhibitor. Further, to examine the effects of Sox4 knockdown on sox2 expression induction by TGF-β, siRNA was stained, and after for 24 hours, was processed for a further 24 hours using TGF-β (100 pM), and the Sox2 mRNA expression levels measured using real-time PCR. The results are shown in Fig. 7D. When Sox4 was knocked down, there was barely any induction of Sox2 expression.

From the above, it was understood that Sox4 was directly induced by TGF-β, and bonded to the Sox2 enhancer region, and up-regulated the expression.

### Implementation Example 7: The Role of Sox4 in Maintaining the Stem Cellularity of .Glioma-Initiating Cells

Until now, there have been absolutely no reports on the relationship of Sox4 to the maintenance of stem cellularity. To examine the role of Sox4 in glioma-initiating cells, the effects of Sox4 knockdown on the stem cellularity of glioma-initiating cells was examined.
The glioma-initiating cells (TGS-01) were separated into single cells, and the Sox4 was knocked down using siRNA, and supplied to either sphere formation assay or limiting dilution assay. The results are shown in Fig. 8A and Fig. 8B. By knocking down Sox4, the sphere formation abilities and self-renewal abilities of the glioma-initiating cells ere reduced. Further, the results of measuring the percentage of CD133-positive cells among the glioma-initiating cells in which Sox4 had been knocked down by siRNA using flow cytometry are shown in Fig. 8C. Knocking down sox4 reduced the percentage of CD133-positive cells (from 72.6% to 41.5% or 41.1%.)
In addition, to examine the effects of Sox4 knockdown on glioma-initiating cell differentiation, the percentage of Nestin-positive cells and GFAP-positive cells among the total cells was measured on the seventh day after the introduction of siRNA. The results are shown in Fig. 8D. It was understood that knocking down Sox4 reduced the Nestin-positive cells, and increased the GFAP-positive cells (see Fig. 8D), and promoted differentiation.

From the results described above, it was understood that Sox4 is involved in an extremely important pathway in the maintenance of the stem cellularity of glioma-initiating cells.

### Implementation Example 8: The Role of Sox4 in Maintaining; the Stem Cellularity of Glioma-Initiating Cells Musing TGF-β

To establish and examine the hypothesis that Sox4 expression, which is induced directly by TGF-β, promotes Sox2 expression and maintains the stem cellularity of glioma-initiating cells, the effects of TGF-β inhibitors on glioma-initiating cells in which Sox4 had been over-expressed were verified. Specifically, the TGS-01 cells were separated into single cells, and stained using adenovirus-sox4 or adenovirus-LacZ, and cultivated for seven days either in the presence or absence of SB431542 (1 µM), and then supplied for sphere formation assay. The results are shown in Fig. 9A. When Sox-4 was caused to over-express in glioma-initiating cells, they showed resistance to SB431542, and the fall in sphere formation ability was alleviated.
Further, to examine the effects of sox4 over-expression on glioma-initiating cell differentiation, the percentage of Nestin-positive cells and GFAP-positive cells among all the cells cultivated for seven days in the same way was measured. The results are shown in Fig. 9B. Even administering the TGF-β inhibitor SB431542, no major changes were observed in the percentages of Nestin-positive cells and GFAP-positive cells. From these results, it was understood that the direct induction of Sox4 expression by TGF-β is indispensable to the maintenance of stem cellularity in glioma-initiating cells.

### Implementation Example 9: The Role of Oct-4 in Maintaining; the Stem Cellularity of Glioma-Initiating Cells

To establish and verify the hypothesis that Oct-4, which is a transcription factor that plays an important role in the maintenance of stem cellularity in ES cells, is involved in the maintenance of stem cellularity in glioma-initiating cells, Oct-4 was knocked down using siRNA.
Both the TGS-01 cells and TGS-04 cells were separated into single cells, and siRNA (siOct-4 No. 1 or siOct-4 No. 2) transfected either to a control (siNC) or Oct-4 and cultured for seven days, and then either sphere formation assay or limiting dilution assay was implemented. The results of the two are shown in Fig. 10A and Fig. 10B respectively. Knocking down Oct-4 conspicuously reduced the sphere formation abilities and self-renewal abilities of the glioma-initiating cells. Further, to examine the effects of Oct-4 knockdown on glioma-initiating cell differentiation, the percentages of Nestin-positive cells, Musashi-positive cells, and GFAP-positive cells among all the cells were measured. The results are shown in Fig. 10C. The percentages of Nestin-positive cells and Musashi-positive cells, which are stem cell markers, decreased, and the percentage of GFAP-positive cells, which is a differentiated cell marker, increased, verifying the fact that differentiation had been promoted.

### Implementation Example 10: The Effects of TGF-β Signal Inhibition In Vivo

To clarify the role of Sox 4 *in vivo* in the maintenance of stem cellularity, the effects of TGF-β inhibitors and Sox4 on the growth of glioma-initiating cells intracranially were examined using intracranial proliferation assay.
Specifically, adenovirus-Sox4 or adenovirus-LacZ staining was implemented, and 5 x 10⁴ TGS-01 cells either unprocessed or after 24 hours processing using a TGF-β inhibitor were transplanted intracranially. TGF-β is well-known to promote glioma cell proliferation, so to classify the results into effects on growth and effects in differentiation, SB431542 processing was implemented as pre-processing and not implemented continuously. Six mice were used for each condition, and evaluated using Kaplan-Meier analysis. The p values were determined using the long-rank test. The results are shown in Fig. 11 (Left). The survival period of the mice to which glioma-initiating cells that had been pre-processed using SB431542 were transplanted was significantly longer than the survival periods of the mice to which the control glioma-initiating cells were transplanted. Further, the results of histological analysis of the samples dissected 30 days after intracranial transplant are shown in Fig. 11 (Right). Tissue sections were stained using hematoxylin and eosin (HE). The mice to which the control glioma-initiating cells were transplanted showed neurological symptoms, and their tumor growth was also large. In contrast, the mice to which glioma-initiating cells that had been pre-processed using SB431542 were transplanted did not show any neurological symptoms, and pathological examination using HE staining did not reveal any tumors. Further, the glioma-initiating cells that over-expressed Sox4 did not shown any effects on extending survival periods using SB431542.

From the above, it was shown that TGF-β controls upwards the expression of Sox2 via the direct induction of Sox4, and that this maintains the stem cellularity of glioma-initiating cells, and that inhibition of the TGF-β-Sox4-Sox2 pathway promotes glioma-initiating cell differentiation. (See Fig. 12.)

### Reference Example 1: Other pathways Involved in the maintenance of Stem Cellularity in Glioma-Initiating Cells

As described above, the inventors showed that the TGF-β-Sox4-Sox2 pathway is indispensable in the maintenance of the stem cellularity of glioma-initiating cells, but the existence *in vivo* of other pathways involved in this is undeniable. For example, it has been reported that the Hedgehog-Gill pathway controls the stem cellularity of glioma-initiating cells, and that cyclopamine, which is a Hedgehog inhibitor, can reduce the mass of glioma tumors. (Clement et al., Current Biology, 2007, 17: 165-172). The mechanism, however, by which inhibition of the Hedgehog-Gill pathway promotes glioma-initiating cell differentiation is unknown.
Here, a sphere formation assay was implemented on TGS-01 cells in the presence of cyclopamine, which is a hedgehog inhibitor. The TGS-01 cells were separated into single cells, and cultured for seven days in the presence of cyclopamine (2.5, 5, and 10 µM; Sigma-Aldrich) and/or SB431542 (1 µM). The results are shown in Fig. 13. Cyclopamine reduced the sphere formation ability of TGS-01 cells, but its effects were smaller than those of SB431542 and, further, even if the two drugs were combined and administered together, no additional or synergistic effect was obtained. From these facts, it is thought that glioma-initiating cells have at least two dependencies: one on the Hedgehog-Gill pathway, and the other on the TGF-β-Sox4-Sox2 pathway. In addition, it has been reported that TGF-β promotes the self-renewal of human glioblastomas through the induction of leukemia inhibitory factor (LIF). (Penuelas et al., Cancer Cell, 2009, 5: 315-327.)
Here, to examine the role of LIF in glioma-initiating cells, TGS-01 and TGS-04 cells were separated into single cells, and cultivated for seven days in the presence of either LIF (20 ng/mL; Chemicon), IgG1 Fc (10 µg/mL) as a control, or anti-LIF neutralizing antibodies (10 µg/mL; R&D Systems). The results are shown in Fig. 14. The sphere formation abilities of cells to which anti-LIF neutralizing antibodies were administered was reduced. TGF-β signals, however, did not induce LIF expression in the TGS-01 or TGS-04 cells. (See Fig. 15.) From these facts, it is thought that TGF-β signals maintain the tumorigenicity of glioma-initiating cells via multiple independent pathways.

### Reference Example 2: Differences of TGF-β Subtypes in Patients

After acidifying the TGS-01 to TGS-05 cells, the TGF-β protein levels secreted in a culture medium were measured using ELISA (R&D Systems). The results are shown in Fig. 16. From the existence of cases in which TGF-β1 was greatly expressed (TGS-05) and cases in which TGF-β2 was greatly expressed (TGS-01, TGS-03, and TGS-04), it has been hinted that treatments targeting only one of the TGF-β subtypes cannot obtain effects depending on the patient.

### Sequence Table Free Text

Sequence Number: 1 is a single-stranded base sequence among the double-stranded RNA that configures siSox2 No. 1.
Sequence Number: 2 is a single-stranded base sequence among the double-stranded RNA that configures siSox2 No. 2
Sequence Number: 3 is a single-stranded base sequence among the double-stranded RNA that configures siSox4 No. 1.
Sequence Number: 4 is a single-stranded base sequence among the double-stranded RNA that configures siSox4 No. 1.
Sequence Number: 5 is a single-stranded base sequence among the double-stranded RNA that configures siSMAD2.
Sequence Number: 6 is a single-stranded base sequence among the double-stranded RNA that configures siSMAD3.
Sequence Number: 7 is a forward primer base sequence used in GAPDH expression analysis.
Sequence Number: 8 is a reverse primer base sequence used in GAPDH expression analysis.
Sequence Number: 9 is a forward primer base sequence used in Sox2 expression analysis.
Sequence Number: 10 is a reverse primer base sequence used in Sox2 expression analysis.
Sequence Number: 11 is a forward primer base sequence used in Sox4 expression analysis.
Sequence Number: 12 is a reverse primer base sequence used in Sox4 expression analysis.
Sequence Number: 13 is a forward primer base sequence used in Oct-4 expression analysis.
Sequence Number: 14 is a reverse primer base sequence used in Oct-4 expression analysis.
Sequence Number: 15 is a forward primer base sequence used in NANOG expression analysis.
Sequence Number: 16 is a reverse primer base sequence used in NANOG expression analysis.
Sequence Number: 17 is a forward primer base sequence used in LIF expression analysis.
Sequence Number: 18 is a reverse primer base sequence used in LIF expression analysis.
Sequence Number: 19 is a forward primer base sequence used in PAI-1 expression analysis.
Sequence Number: 20 is a reverse primer base sequence used in PAT-1 expression analysis.
Sequence Number: 21 is a forward primer base sequence used in p15INK4b expression analysis.
Sequence Number: 22 is a reverse primer base sequence used in p15INK4b expression analysis.
Sequence Number: 23 is a forward primer base sequence used in p21WAF1 expression analysis.
Sequence Number: 24 is a reverse primer base sequence used in p21WAFl expression analysis.
Sequence Number: 25 is a forward primer base sequence used in p27KIP1 expression analysis.
Sequence Number: 26 is a reverse primer base sequence used in p27KIP1 expression analysis.
Sequence Number: 27 is a forward primer base sequence used in c-Myc expression analysis.
Sequence Number: 28 is a reverse primer base sequence used in c-Myc expression analysis.
Sequence Number: 29 is a forward primer base sequence used in TGF-β1 expression analysis.
Sequence Number: 30 is a reverse primer base sequence used in TGF-β1 expression analysis.
Sequence Number: 31 is a forward primer base sequence used in TGF-β2 expression analysis.
Sequence Number: 32 is a reverse primer base sequence used in TGF-β2 expression analysis.
Sequence Number: 33 is a forward primer base sequence used in TGF-β3 expression analysis.
Sequence Number: 34 is a reverse primer base sequence used in TGF-β3 expression analysis.
Sequence Number: 35 is a forward primer base sequence used in TGF-β type II receptor expression analysis.
Sequence Number: 36 is a reverse primer base sequence used in TGF-β type II receptor expression analysis.
Sequence Number: 37 is a forward primer base sequence used in ALK5 expression analysis.
Sequence Number: 38 is a reverse primer base sequence used in ALK5 expression analysis.
Sequence Number: 39 is a forward primer base sequence used in SMAD2 expression analysis.
Sequence Number: 40 is a reverse primer base sequence used in SMAD2 expression analysis.
Sequence Number: 41 is a forward primer base sequence used in SMAD3 expression analysis.
Sequence Number: 42 is a reverse primer base sequence used in SMAD3 expression analysis.
Sequence Number: 43 is a forward primer base sequence used in SMAD4 expression analysis.
Sequence Number: 44 is a reverse primer base sequence used in SMAD4 expression analysis.
Sequence Number: 45 is a forward primer base sequence used in HPRT1 PCR during immunoprecipitation.
Sequence Number: 46 is a reverse primer base sequence used in HPRT1 PCR during immunoprecipitation.
Sequence Number: 47 is a forward primer base sequence used in Sox4 PCR during immunoprecipitation.
Sequence Number: 48 is a reverse primer base sequence used in Sox4 PCR during immunoprecipitation.
Sequence Number: 49 is a forward primer base sequence used in Sox2 PCR during immunoprecipitation.
Sequence Number: 50 is a reverse primer base sequence used in Sox2 PCR during immunoprecipitation.
Sequence Number: 51 is a single-stranded base sequence among the double-stranded RNA that configures siOct-4 No. 1.
Sequence Number: 52 is a single-stranded base sequence among the double-stranded RNA that configures siOct-4 No. 2.

## Claims

1. A promoter for differentiation of brain tumor initiating cells comprising a substance that inhibits a TGF-β-Sox4-Sox2 pathway in a brain tumor initiating cell.

2. The promoter for differentiation of brain tumor initiating cells of claim 1, wherein the substance that inhibits the TGF-β-Sox4-Sox2 pathway is an expression inhibitor or function inhibitor against at least one gene selected from the group consisting of Sox2, Sox4, TGF-β, SMAd2, SMAd3, and Oct-4.

3. The promoter for differentiation of brain tumor initiating cells of claim 2, wherein the expression inhibitor or function inhibitor is a TGF-β receptor antagonist.

4. The promoter for differentiation of brain tumor initiating cells of claim 2, wherein the expression inhibitor or function inhibitor is a compound selected from the group consisting of:
(a) an antisense nucleic acid against at least one gene selected from the group consisting of Sox2, Sox4, SMAd2, SMAd3, and Oct-4,
(b) a nucleic acid having ribozyme activity against at least one gene selected from the group consisting of Sox2, Sox4, SMAd2, SMAd3, and Oct-4,
(c) a double-stranded nucleic acid having an RNAi effect against at least one gene selected from the group consisting of Sox2, Sox4, SMAd2, SMAd3, and Oct-4, and
(d) a nucleic acid that encodes the nucleic acid described in any of (a) and (c).

5. The promoter for differentiation of brain tumor initiating cells of any one of claims 1 to 4, wherein the promoter for differentiation of brain tumor initiating cells is a promoter for differentiation of glioma initiating cells.

6. A brain tumor therapeutic agent comprising a promoter for differentiation of brain tumor initiating cells of any one of claims 1 to 4.

7. The brain tumor therapeutic agent of claim 6, wherein the brain tumor therapeutic agent is a glioma therapeutic agent.

8. A double-stranded nucleic acid having a RNAi effect against Sox2, the double-stranded nucleic acid consisting of two nucleic acids of either:
(i) a nucleic acid consisting of the base sequence as set forth in SEQ ID NO:1 and a nucleic acid consisting of the base sequence complementary to such nucleic acid; or
(ii) a nucleic acid consisting of the base sequence as set forth in SEQ ID NO:2 and a nucleic acid consisting of the base sequence complementary to such nucleic acid.

9. A double-stranded nucleic acid having a RNAi effect against Sox4, the double-stranded nucleic acid consisting of two nucleic acids of either:
(i) a nucleic acid consisting of the base sequence as set forth in SEQ ID NO:3 and a nucleic acid consisting of the base sequence complementary to such nucleic acid; or
(ii) a nucleic acid consisting of the base sequence as set forth in SEQ ID NO:4 and a nucleic acid consisting of the base sequence complementary to such nucleic acid.

10. A double-stranded nucleic acid having a RNAi effect against Smad2, the double-stranded nucleic acid consisting of a nucleic acid consisting of the base sequence as set forth in SEQ ID NO:5 and a nucleic acid consisting of the base sequence complementary to such nucleic acid.

11. A double-stranded nucleic acid having a RNAi effect against Smad3, the double-stranded nucleic acid consisting of a nucleic acid consisting of the base sequence as set forth in SEQ ID NO:6 and a nucleic acid consisting of the base sequence complementary to such nucleic acid.

12. A double-stranded nucleic acid having a RNAi effect against Oct4, the double-stranded nucleic acid consisting of two nucleic acids of either:
(i) a nucleic acid consisting of the base sequence as set forth in SEQ ID NO:51 and a nucleic acid consisting of the base sequence complementary to such nucleic acid; or
(ii) a nucleic acid consisting of the base sequence as set forth in SEQ ID NO:52 and a nucleic acid consisting of the base sequence complementary to such nucleic acid.

13. A method for treating a brain tumor, the method comprising the steps of:
collecting tissues from a site of a patient affected by a brain tumor to measure expression level of Sox2 and/or Sox4 in the tissues; and
administering the brain tumor therapeutic agent of claim 6 or 7 when the expression level of Sox2 and/or Sox4 is increased in comparison with that in normal tissues.

14. A screening method for determining a treatment method for a patient with a brain tumor, the method comprising the steps of:
collecting tissues from the site of a patient affected by a brain tumor to measure expression level of Sox2 and/or Sox4 in the tissues; and
comparing the expression level with expression level in normal tissues.
